(19) **Europäisches Patentamt / European Patent Office / Office européen des brevets**

(11) **EP 4 164 478 B1**

(12) # EUROPEAN PATENT SPECIFICATION

(45) Date of publication and mention of the grant of the patent:
**08.05.2024 Bulletin 2024/19**

(21) Application number: **21732579.4**

(22) Date of filing: **11.06.2021**

(51) International Patent Classification (IPC):
*A61B 5/024* (2006.01)   *A61B 5/11* (2006.01)
*A61B 5/16* (2006.01)   *A61B 5/00* (2006.01)

(52) Cooperative Patent Classification (CPC):
**A61B 5/165; A61B 5/024; A61B 5/1118; A61B 5/4866; A61B 5/681**

(86) International application number:
**PCT/IB2021/055168**

(87) International publication number:
**WO 2021/250630 (16.12.2021 Gazette 2021/50)**

(54) **WEARABLE DETECTION SYSTEM FOR DETECTING VULNERABILITY FOR AND INFECTION OF A HOMEOTHERMIC LIVING ORGANISM**

WEARABLE-DETEKTIONSSYSTEM ZUR DETEKTION DER ANFÄLLIGKEIT FÜR UND DER INFEKTION MIT EINEM HOMÖOTHERMEN LEBENDEN ORGANISMUS

SYSTÈME DE DÉTECTION VESTIMENTAIRE POUR DÉTECTER LA VULNÉRABILITÉ ET L'INFECTION D'UN ORGANISME VIVANT HOMÉOTHERME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.06.2020 EP 20179581**

(43) Date of publication of application:
**19.04.2023 Bulletin 2023/16**

(73) Proprietor: **BioRICS NV**
**3001 Leuven (BE)**

(72) Inventors:
• **BERCKMANS, Daniel**
**3001 Leuven (Heverlee) (BE)**
• **PENA FERNANDEZ, Alberto**
**3001 Leuven (Heverlee) (BE)**

(74) Representative: **Callewaert, Raf et al**
**Bureau Callewaert**
**Brusselsesteenweg 108**
**3090 Overijse (BE)**

(56) References cited:
**WO-A1-2008/003148    WO-A1-2015/193514**

• **Anonymous: "Wearables like Fitbit and Oura can detect coronavirus symptoms, new research shows - The Washington Post", , 28 May 2020 (2020-05-28), XP055741552, Retrieved from the Internet: URL:https://www.washingtonpost.com/technology/2020/05/28/wearable-coronavirus-detect / [retrieved on 2020-10-19]**

**Description**

FIELD OF THE INVENTION

The invention concerns a detection system for detecting vulnerability or risk for infection and/or inflammation and by using this prediction to realise an early and accurate detection of infection and/or inflammation of a homeothermic living organism. The system comprises at least one sensor to measure and monitor heart rate and physical activity of the living organism, at least one processor to process the measured heart rate and physical activity, and at least one output unit to generate a result, an advice or an alert.

BACKGROUND OF THE INVENTION

As shown in history the risk for infections or a foreign body -such as bacteria, virus, and parasites- into the body of human or an animal is a reality. The last pandemic in 2018 did cost life to around 50 M people. The African Swine Fever (ASF) in 2019 did end up with the loss of about 40 % of all pigs in the world. In some cases, the transition of diseases and the infection goes while the infected subject is not aware (yet) of being infected (e.g., humans are not sensitive for ASF, but they transport and distribute it in pig meat in the food). The worst case in the modern global and fast travelling world is when an infectious disease is transferred before showing symptoms in the infected person or animal as it seems to the case with the COVID-19 Corona virus. A solution that offers a detection of infection, even before symptoms are shown, can bring high added value to the world.

The immune system comprises cells and organs whose functions can be overgeneralized as first to recognize non self-entities such as viruses, bacteria and parasites in the body and next to destroy them. These two aims are accomplished via a complex regulatory network of organs, cells, cell receptors and proteins. Scientific evidence demonstrates that the immune system is highly integrated with the nervous system. Stressful events reliably associate with changes in the immune system (10). Since Selye's (1975) finding of an initial model in which stress is broadly immunosuppressive, conceptualizations of the nature of the relationship between stress and the immune system have changed over time (11, 12). Since then a vast amount of scientific papers report on a relationship between psychological or mental stress and parameters of the immune system in human participants while the flexibility of the immune system can be compromised by physical condition, age and disease.

A generic immune response reaction to protect the body from infection, i.e. foreign bodies or non self-entities in the body, is inflammation. However, in some cases inflammation is not caused by infection, but other causes such as e.g. autoimmune diseases, inflammatory diseases and/or tissue damage.

Whatever physical or mental performance, done by a living organism, requires metabolic energy. The metabolic energy, produced by the body of an individual, is used for different components: e.g. keeping organs functioning (the basal component including the immune system), the circadian basal component at minimal physical and mental performance, control of body temperature, physical activity, mental activity and growth or production in livestock (e.g. meat, milk, eggs). It is logic that the amount of metabolic energy, used for the physical component during physical activities or for the mental component during stressful events, is not available anymore for one of the other components and among them the immune system. Moreover, a negative stressor is alarming the body to go into fly or flight mode and is meanwhile depressing the immune system to save metabolic energy.

Each individual requires a minimal amount of metabolic energy to keep organs functioning, which needs a minimal heart rate. The minimal required heart rate is often called the resting heart rate (RHR). An increase of the resting heart rate is indicative for infection since the immune system is asking for more energy. However, the bioenergetic system related to infection is much more complex than just an increase of resting heart rate. To go from resting heart rate to early warning and accurate detection of infection is not trivial at all.

When the individual is sleeping in a horizontal position, the required energy is minimal and the resting heart rate (RHR) may be measured. During sleeping there are no physical performances. However, it is also assumed that there are no mental loads, which is not known. Possibly there is a (chronic) mental stressor or the individual is dreaming.

When the individual is awake and mentally relaxing with a minimal physical performance, not in a horizontal position, the minimal required heart rate will be higher than when sleeping during the night. According to the state of the art, this heart rate level is called the (Daily) Resting Heart Rate (DRHR). Mostly it is measured after a short rest period without physical activity. An accelerometer on a wearable may determine whether the individual is at rest and has not recently been moving within e.g. a previous 5 minutes window. As such, it is prevented that a physical component of heart rate is present when measuring the daily resting heart rate. However, it is not possible to exclude presence of a mental component of heart rate when measuring the resting heart rate in this way.

At present detection of infection is attempted by analysing (Daily) Resting Heart Rates, which are analysed and evaluated by using thresholds based on statistical population data. However, each individual immune system has its own history resulting in a diversity of totally different individual immune responses and even within the same individual, it is varying over time when new immune challenges are experienced.

As such current systems and methods using Daily Resting Heart Rate are not good enough to (i) early detect infection of an individual and (ii) to accurately detect an infection in terms of true and false positives and true and false negatives.

As said by Prof. Snyder (Stanford, 2020) "The bottom line is it is working, but it's not perfect" and we are yet away from a reliable product.

SUMMARY OF THE INVENTION

The main objective of the current invention is to propose a device, that is preferably wearable for (i) detecting vulnerability to infectious diseases such as viral infection, bacterial infection, and/or inflammation (ii) for early detection of actual infections and/or inflammation possibly even before regular symptoms such as fever occur and (iii) for accurate detection of infections and/or inflammation in terms of true and false positives and negatives. This is done by continuously measuring physical activity and heart rate and further monitoring and decomposing heart rate in the different components related to metabolic energy use and evaluating energy expenditure versus recovery.

The solution of the current invention offers a detection system that monitors vulnerability or risk for infection resulting in prediction of infection and further generates a detection of infection. The present invention may possibly also detect inflammation that is not necessarily caused by infection. The objective of monitoring of vulnerability for infection, or also risk for infection and/or inflammation, is a prediction of possible infection and/or inflammation and has the advantage that it allows that measures can be taken to reduce the infection risk and possibly to prevent an infection and/or inflammation.

It is further an object of the invention to propose a device, that preferably continuously measures and monitors balance between metabolic energy use, for physical and mental energy use, versus recovery over time for generating an overview of individual mental energy use versus recovery, which may also be referred to as the stress/recovery balance. The energy use involves the different components in the homoeothermic energy system: the basal component to keep organs functioning, the circadian basal component when the individual is awake with minimal physical efforts and mentally relaxing, the physical component during physical performances and the mental component due to stress, cognitive load or e.g. happiness.

The invention works continuously on moving subjects during full activity and movements.

From continuous monitoring over longer periods, it shows that long periods with shortage of recovery lead to vulnerability for infections and/or inflammation.

It is further an object of the invention to propose an individual adaptive threshold to detect infection and/or inflammation of the individual.

The above-mentioned objects are realised by the method and device having the specific features set out in the appended claims. Specific features for preferred embodiments of the invention are set out in the dependent claims.

Practically, the detection system according to the invention comprise at least one processor programmed to

- decompose the heart rate in at least time series of a physical heart rate component due to physical activity ($HR_{physical}$), time series of a mental heart rate component due to mental activity ($HR_{mental}$) and time series of a circadian basal heart rate component due to circadian basal metabolism ($HR_{circadian}$);
- calculate comparative individual levels of metabolic energy use for the physical activity and the mental activity based on preceding time series of, respectively, at least the physical and the mental heart rate components;
- calculate current individual levels of metabolic energy use for the physical activity and the mental activity based on current heart rate components;
- determine recovery when the current individual level of metabolic energy use is lower than the comparative individual level of metabolic energy use for the physical activity and the mental activity;
- determine load when the current individual level of metabolic energy use is higher than the comparative individual level of metabolic energy use for the physical activity and the mental activity;
- calculate resilience based on the ratio between the recovery and the load for the physical activity and the mental activity;
- compare current resilience with at least one resilience threshold for the physical activity and the mental activity and determine whether the at least one resilience threshold has been reached for the physical activity and/or the mental activity;
- compare current circadian basal heart rate component with at least one circadian basal heart rate threshold and determine whether the at least one circadian basal heart rate threshold has been reached.

The detection system further comprise an output unit configured to generate at least one result, preferably an alert that comprises a detection warning, i.e. an infection warning or a detection of infection and/or inflammation, when the at least one processor determines that the at least one resilience threshold has been reached and the at least one circadian basal heart rate threshold has been reached.

The output unit may comprise a display, a printer, a sound generator for generating an audible alert, a signal transmitter for transmitting the result to a remote recording system. The result, such as the detection of infection, could be transmitted together with a location signal, such as GPS coordinates, for tracking and monitoring infection outbreak in a population.

According to an embodiment of the detection system, the at least one result comprises a vulnerability warning when the processor determines that the at least one resilience threshold has been reached for the physical activity and/or the mental activity. The vulnerability warning concerns a risk for infection and/or inflammation and is hence a prediction of possible infection and/or inflammation.

According to an embodiment of the detection system, the comparative individual levels of metabolic energy use are levels obtained from preceding time series of the heart rate components, preferably over a previous time window of about 2 to 60 days, more preferably a period of 10 to 40 days, in particular a period of about one month. As such, the level obtained from preceding time series may be obtained from a moving average, a trend, a median, a smoothed interpolation or a spline function.

According to a preferred embodiment of the detection system, the at least one resilience threshold comprise at least one mental resilience threshold for the mental resilience and at least one physical resilience threshold for the physical resilience, and the output unit is configured to generate the result when the processor determines that the at least one mental resilience threshold has been reached and/or the at least one physical resilience threshold has been reached.

According to the preferred embodiment of the detection system, comparing current resilience with at least one resilience threshold for the physical activity and the mental activity comprise comparing current resilience with at least one preceding resilience and determining whether the at least one resilience threshold has been reached for the physical activity and the mental activity.

Preferably, comparing current resilience with preceding resilience comprise subtracting at least one preceding resilience from the current resilience and determining that the at least one resilience threshold has been reached when the at least one preceding resilience subtracted from the current resilience is lower than the at least one resilience threshold.

Advantageously, the at least one preceding resilience comprise an average of preceding resilience of a number of preceding timeframes and the at least one resilience threshold comprise a long term resilience threshold, whereby the at least one processor is further programmed to compare the current resilience with said average resilience and to determine whether the long term resilience threshold has been reached, and whereby the output unit is further configured to generate the result when the processor determines that the long term resilience threshold has been reached.

A timeframe may correspond to a period of one day and the average of preceding resilience may be obtained from a number of preceding timeframes corresponding to a total period of one week, one month or several months.

Moreover, the at least one resilience threshold may further comprise a short term resilience threshold and the at least one preceding resilience may further comprise an immediately preceding resilience of an immediately preceding timeframe, whereby the at least one processor is further programmed to compare the current resilience with the immediately preceding resilience and to determine whether the short term resilience threshold has been reached, and whereby the output unit is further configured to generate the result when the processor determines that both the short term resilience threshold and the long term resilience threshold have been reached.

According to an embodiment of the detection system the at least one processor is further programmed to calculate comparative individual levels of metabolic energy use for the circadian basal metabolism based on preceding time series of the circadian basal heart rate heart rate for use as the at least one circadian basal heart rate threshold.

According to the preferred embodiment of the detection system the at least one processor is further programmed to analyse dynamics of the circadian basal heart rate component by comparing the current circadian basal heart rate component with at least one preceding circadian basal heart rate component of at least one preceding timeframe and determining whether the at least one circadian basal heart rate threshold has been reached.

Preferably, the at least one processor is thereby further programmed to analyse dynamics of the circadian basal heart rate component by

- comparing the current circadian basal heart rate component with the immediately preceding circadian basal heart rate component of an immediately preceding timeframe and a fast circadian basal heart rate threshold of the at least one circadian basal heart rate threshold; and

- comparing the current circadian basal heart rate component with an average preceding circadian basal heart rate component of a number of preceding timeframes and a slow circadian basal heart rate threshold of the at least one circadian basal heart rate threshold;

and the output unit is further configured to generate the result when the processor also determines that the fast circadian basal heart rate threshold and the slow circadian basal heart rate threshold have been reached.

More preferably, the at least one processor is thereby further programmed to analyse dynamics of the circadian basal heart rate component by comparing with the at least one threshold

- a fast trend of the circadian basal heart rate component, corresponding to a change over a short period of 1 to 2 days, and
- a slow trend of the circadian basal heart rate component, corresponding to a change over a long period of 10 to 40 days.

According to an embodiment of the detection system, decomposing the heart rate comprise using a state-space representations model.

According to an embodiment of the detection system, decomposing the heart rate comprise estimating the physical heart rate component, the mental heart rate component and the circadian basal heart rate component in real-time.

According to an embodiment of the detection system, the at least one sensor comprises an accelerometer, a gyroscope, a motion sensor, a GPS, a camera, an electrical sensor, an optical sensor, an electrocardiogram device, a heart sound sensor, a laser device, a magnetic field sensor, a pedometer and/or a sound analyser.

According to an embodiment of the detection system, it is at least partially integrated in a wearable device such as a smartwatch, smartphone, breast band, bracelet, patch and/or sticker.

According to an embodiment of the detection system, resilience is calculated in a timeframe of at least one day and the current resilience is the resilience of at least the current day.

Preferably the timeframe corresponds to a period of at least one day and the number of preceding timeframes corresponds to a total period of 3 to 60 days, in particular a period of about one week or about one month.

According to an interesting embodiment of the detection system, the at least one processor is further programmed to

- calculate comparative individual levels of metabolic energy use for the circadian basal metabolism based on preceding time series of the circadian basal heart rate heart rate component;
- calculate current individual levels of metabolic energy use for the circadian basal metabolism based on the current circadian basal heart rate component;
- determine recovery when the current individual level of metabolic energy use is lower than the comparative individual level of metabolic energy use for the circadian basal metabolism; and
- determine load when the current individual level of metabolic energy use is higher than the comparative individual level of metabolic energy use for the circadian basal metabolism;

for classifying the detection warning as a bacterial infection warning or as a viral infection warning, whereby the at least one processor is still further programmed to

- classify the detection warning as a bacterial infection warning when the processor determines that the metabolic energy use for the circadian basal metabolism increases between 5 and 30 days, preferably between 10 and 25 days, before the detection warning and at the moment of the detection warning; and/or
- classify the detection warning as a viral infection warning when the processor determines that the recovery for the circadian basal metabolism increases between 2 and 9 days, preferably between 5 to 6 days, before the detection warning and the metabolic energy use for the circadian basal metabolism increases between 5 and 15 days, preferably about 10 day, after the detection warning;

whereby the output unit is further configured to generate the at least one result that comprises a viral infection warning and/or a bacterial infection warning.

The invention also relates to a computer readable medium storing a computer program and instructions for performing a method for predicting vulnerability and detecting infection and/or inflammation of a homeothermic living organism, the method comprising:

- measuring and monitoring, with at least one sensor, as function of time heart rate and physical activity of the living organism for obtaining time series of heart rate and physical activity;
- decomposing, with at least one processor, the heart rate in at least time series of a physical heart rate component due to physical activity ($HR_{physical}$), time series of a mental heart rate component due to mental activity ($HR_{mental}$) and time series of a circadian basal heart rate component due to basal metabolism ($HR_{circadian}$);
- calculating, with the at least one processor, comparative individual levels of metabolic energy use based on preceding time series of at least one of the heart rate components;
- calculating, with the at least one processor, current individual levels of metabolic energy use based on current heart rate components;

- determining, with the at least one processor, recovery when the current individual level of metabolic energy use is lower than the comparative individual level of metabolic energy use;
- determining, with the at least one processor, load when the current individual level of metabolic energy use is higher than the comparative individual level of metabolic energy use;
- calculating, with the at least one processor, resilience based on the ratio between the recovery and the load;
- comparing, with the at least one processor, current resilience with preceding resilience and determine whether at least one resilience threshold has been reached;
- comparing, with the at least one processor, current circadian basal heart rate component with at least one circadian basal heart rate threshold;
- generating a result by an output unit when the processor determines that the at least one resilience threshold and the at least one circadian basal heart rate threshold have been reached.

Other particularities and advantages of the invention will become clear from the following description and accompanying drawings; the description and drawings are given as an example only and do not limit the scope of the claimed protection in any way.

DESCRIPTION OF THE DRAWINGS

Figure 1 is an example of a general working scheme of a detection system according to the invention to monitor vulnerability and to detect infection and/or inflammation by using wearables.

Figure 2 is a schematic overview of a general method for operating the detection system according to the invention.

Figure 3 is an overview of 5 main steps in a possible algorithm used by the detection system according to the invention to generate an alert of vulnerability for infection and/or inflammation (prediction alert) and further an alert of infection and/or inflammation (detection alert).

Figure 4 is a possible scheme of the method wherein the total heart rate and the physical activity, e.g. movement, are measured for obtaining the circadian basal ($HR_{circadian}$), the physical ($HR_{physical}$) and the mental ($HR_{mental}$) components of heart rate.

Figure 5 is a possible scheme of an algorithm to decompose the total heart rate in the basal ($HR_{basal}$), the circadian basal ($HR_{circadian}$), the physical ($HR_{physical}$) and the mental ($HR_{mental}$) components of heart rate.

Figure 6 is a representation of the resulting components of total heart rate decomposed by the algorithm of figure 5 in real-time in the circadian basal ($HR_{circadian}$), the physical ($HR_{physical}$) and the mental ($HR_{mental}$) components.

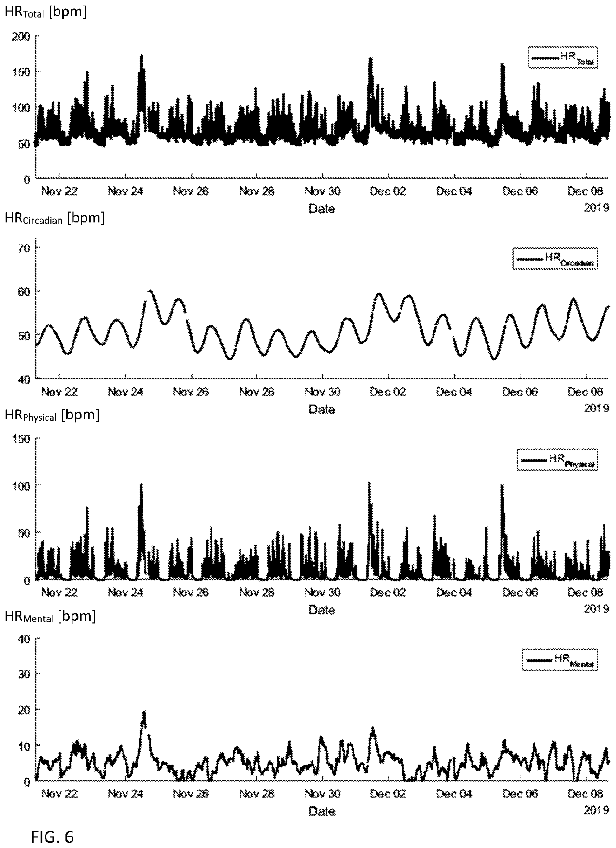

Figure 6 is a schematic representation of the difference during one day between the level of resting heart rate (RHR), as a daily constant value, and the basal heart rate ($HR_{basal}$) and circadian basal heart rate ($HR_{circadian}$) obtained according to the current invention.

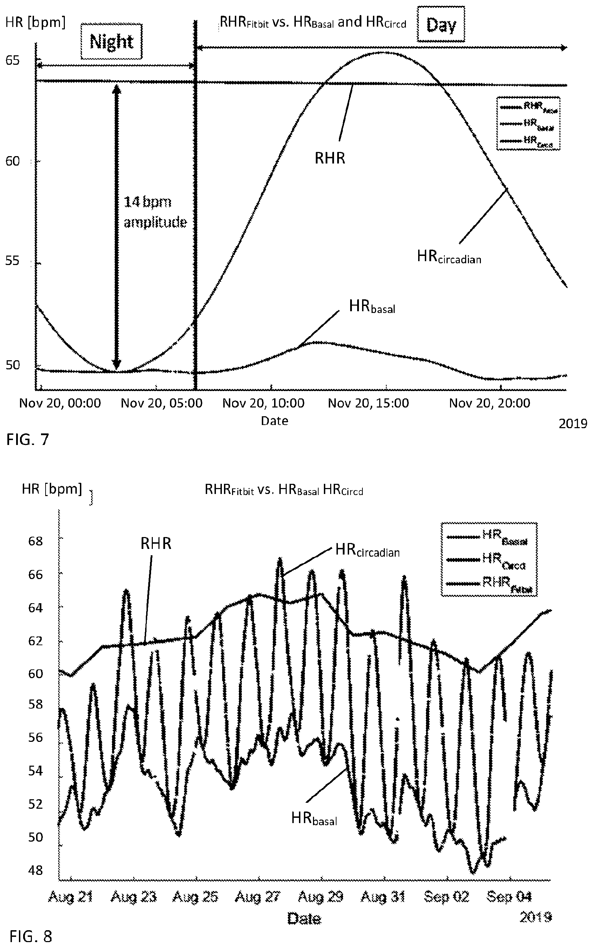

Figure 7 is a schematic representation as in figure 6 for a period of 14 days.

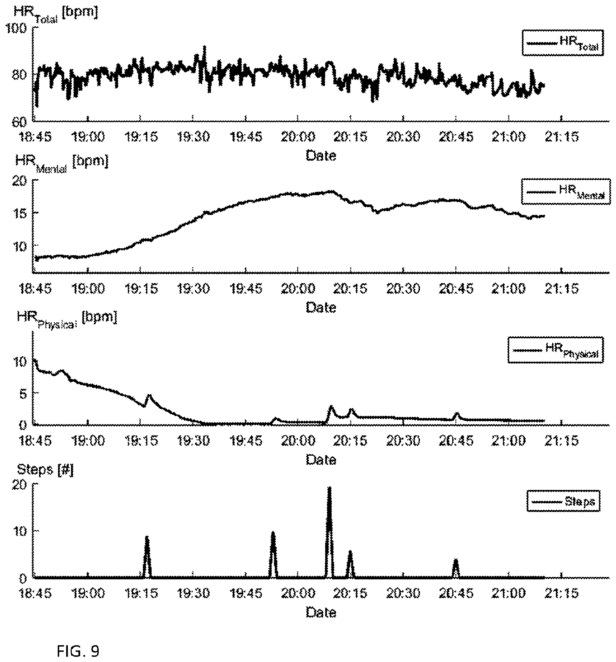

Figure 9 is an example of the difference between the total heart rate ($HR_{total}$), the physical heart rate ($HR_{physical}$) and the mental heart rate ($HR_{mental}$) during an active day over a few hours and the corresponding physical activity as a number of steps.

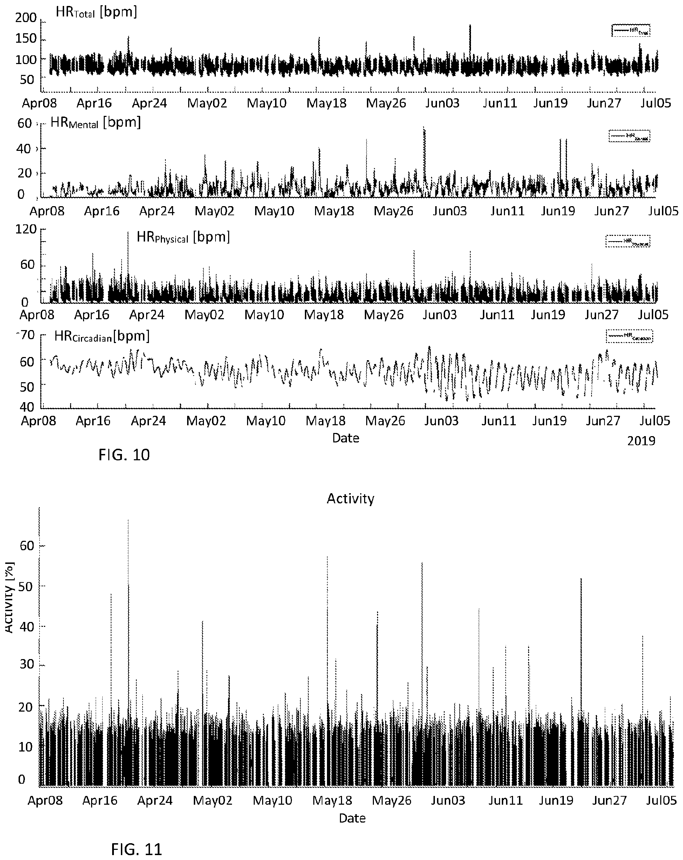

Figure 10 is an example as is figure 9 over a 3 months period.

Figure 11 is the corresponding physical activity measured during the period of figure 6B.

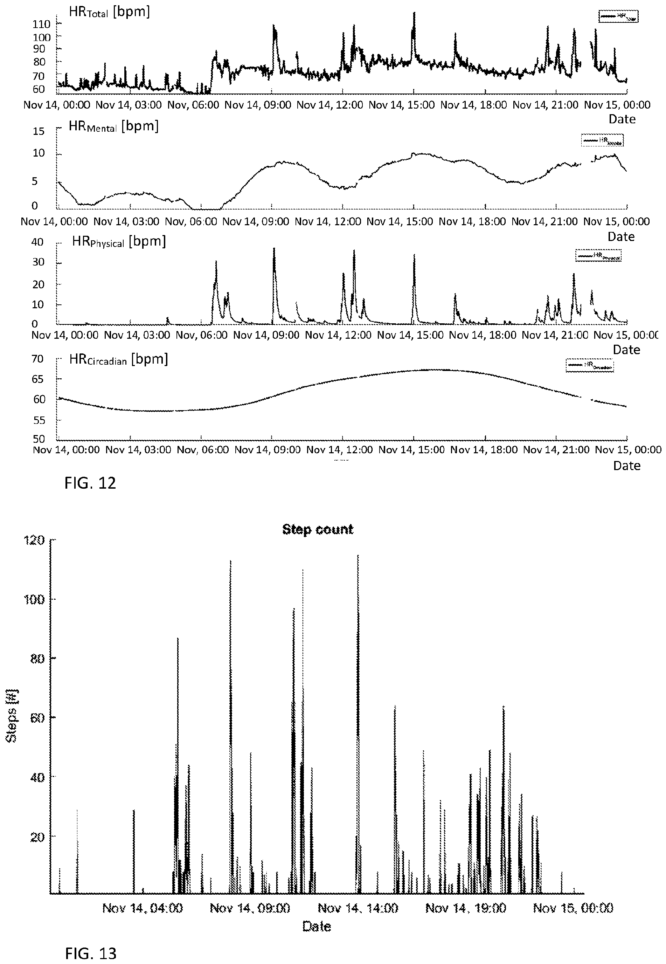

Figure 12 is an example of a decomposition result of the total heart rate ($HR_{total}$) in the circadian basal ($HR_{circadian}$), the physical ($HR_{physical}$) and the mental ($HR_{mental}$) components over a period of 24 hours.

Figure 13 is the corresponding physical activity as a number of steps measured during the period of figure 12.

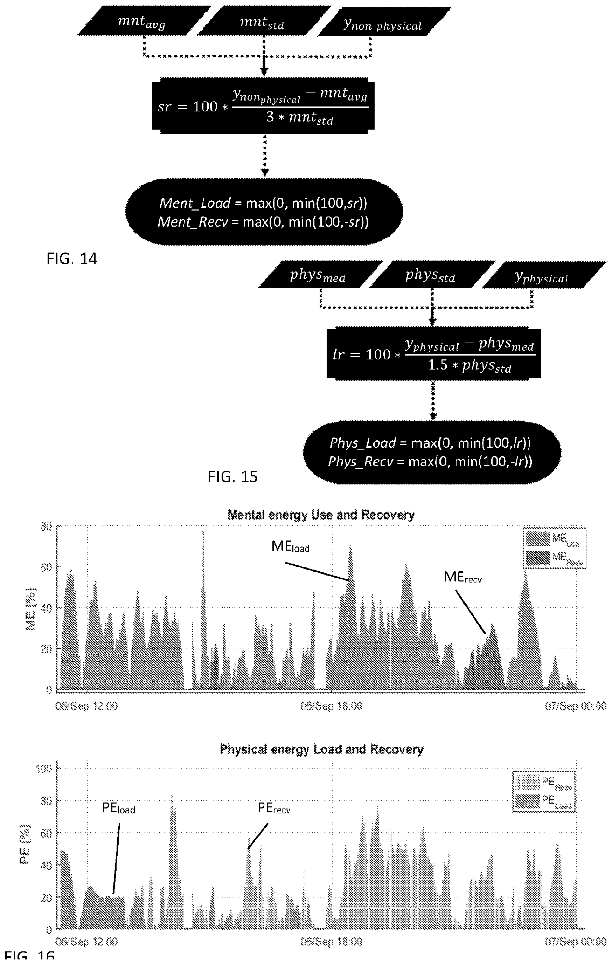

Figure 14 is a scheme of a possible algorithm according to the invention to calculate load and recovery for the mental activity component.

Figure 15 is a scheme of a possible algorithm according to the invention to calculate load and recovery for the physical activity component.

Figure 16 is an example of a result of the algorithms of figures 14 and 15 with real-time information on energy use and/or recovery for the mental (upper graph, mental energy use or also called load (*Ment_Load* / $ME_{load}$), mental recovery (*Ment_Recv* / $ME_{recv}$)) and for the physical component (lower graph, physical energy use or also called load (*Phys_Load* / $PE_{load}$), physical recovery (*Phys_Recv* / $PE_{recv}$)).

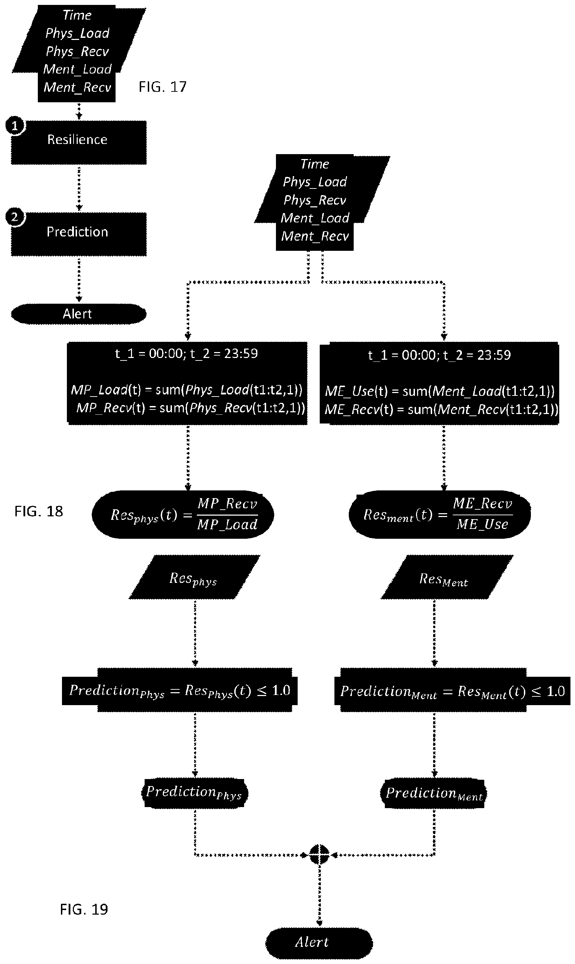

Figure 17 is an overview of a possible general algorithm according to the invention to generate the alert of vulnerability for infection and/or inflammation.

Figure 18 is a scheme of a possible algorithm according to the invention to calculate physical and mental resilience.

Figure 19 is a scheme of a possible algorithm according to the invention to evaluate mental and physical resilience to generate the alert of vulnerability for infection and/or inflammation.

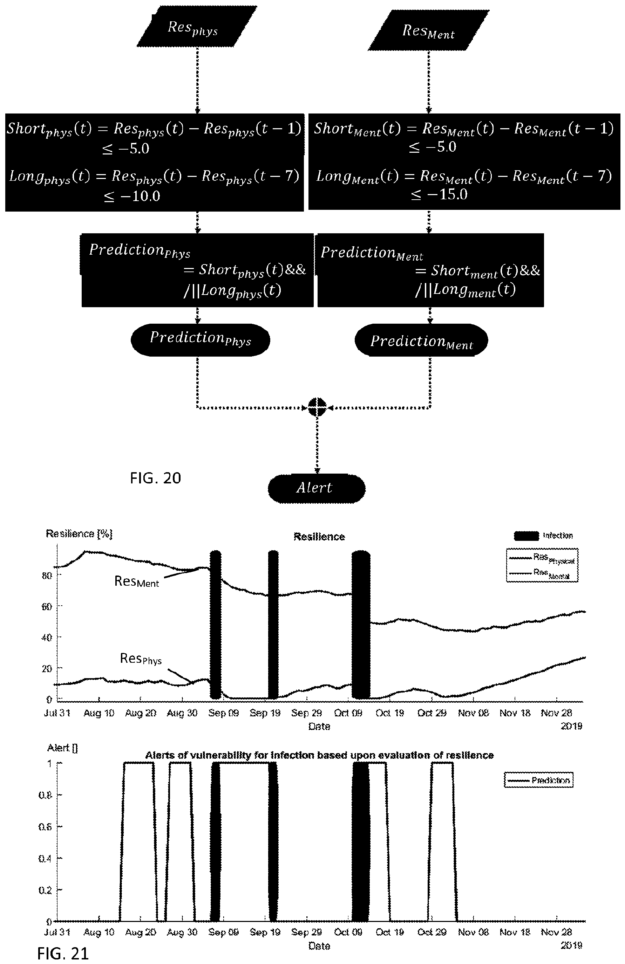

Figure 20 is a scheme of another possible algorithm according to the invention to evaluate mental and physical resilience to generate the alert of vulnerability for infection and/or inflammation.

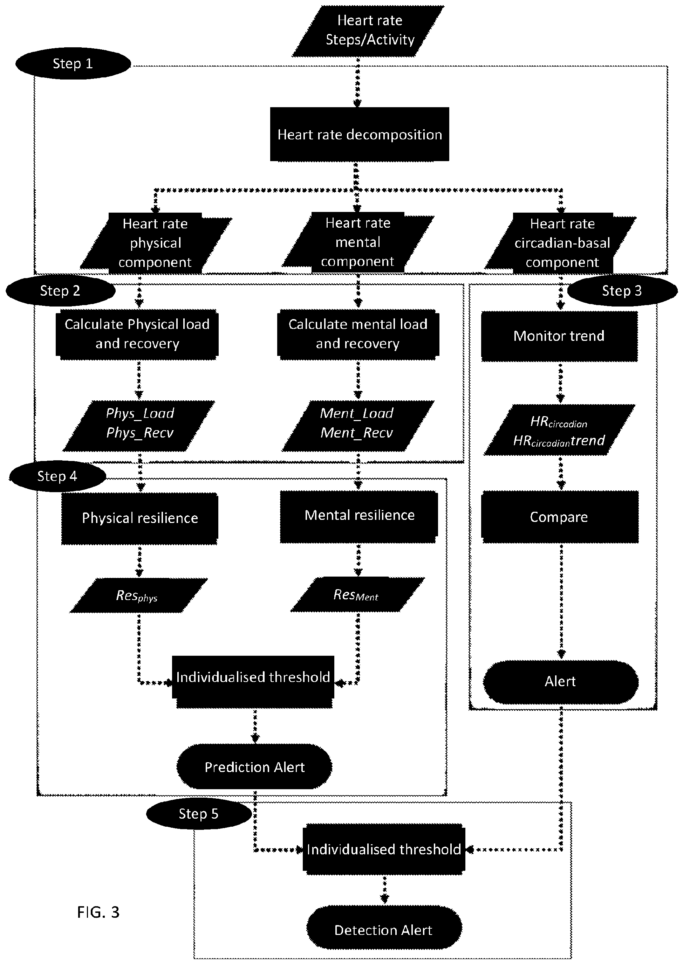

Figure 21 is an example of the evolution of resilience and the detection of vulnerability resulting from the algorithms of figures 3, 5, 14, 15, 17, 18, 19 and further the detection of infection and/or inflammation and the generation of alerts of vulnerability for infection and/or inflammation and alerts of infection and/or inflammation.

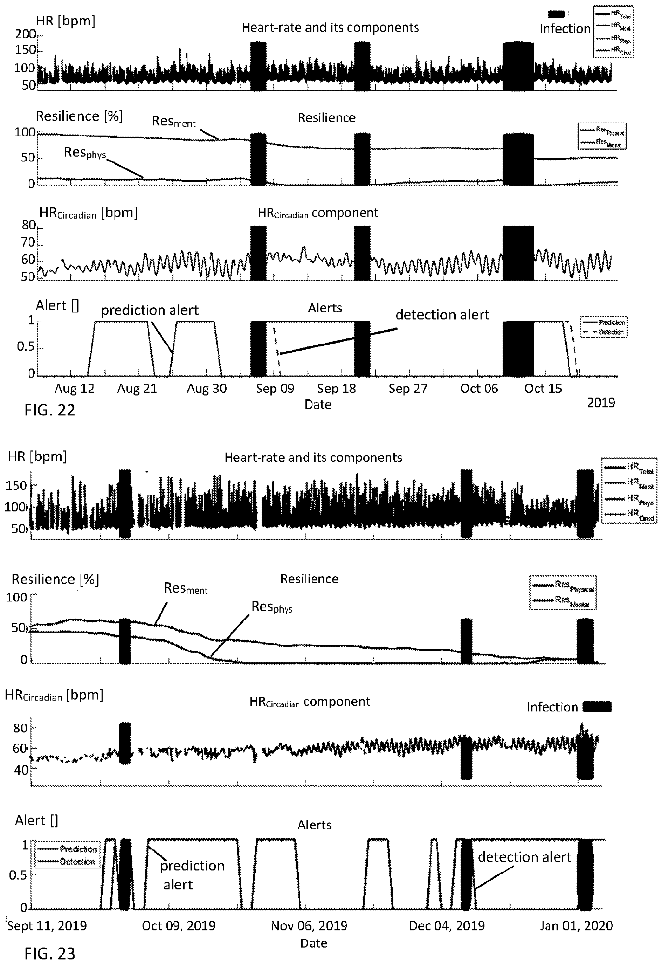

Figure 22 is an example of several alerts of vulnerability for infection (prediction alert) and an alert for infection (detection alert) according to a detection system of the invention, the alerts being based on the combination of evaluation of the evolution of physical resilience ($Res_{phys}$) and mental resilience ($Res_{ment}$) and evaluation of the evolution of circadian basal heart rate component ($HR_{circadian}$).

Figure 23 is another example of a resulting prediction of possible infection by alert of vulnerability (prediction alert) and later alert for infection (detection alert).

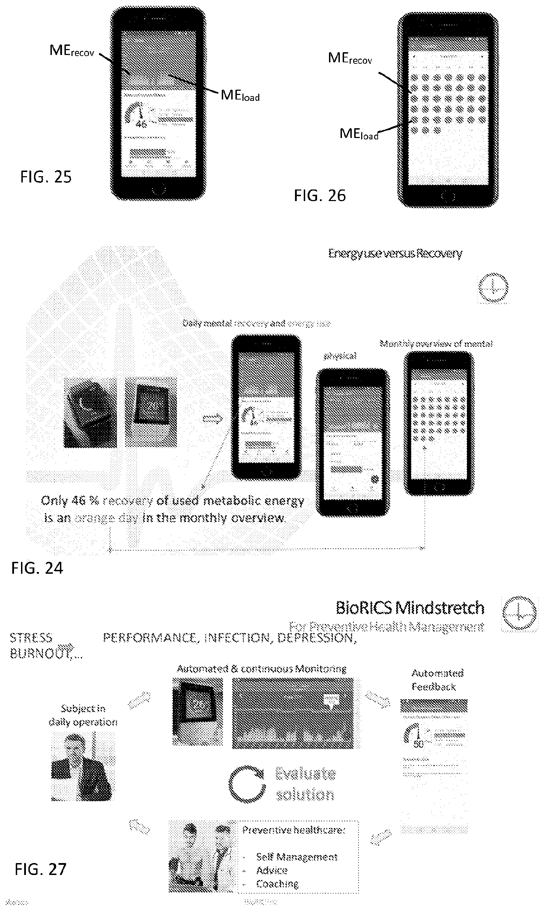

Figure 24 is an example of sensor, processor and display as in the Mindstretch product using a smartwatch and/or a smartphone.

Figure 25 is an example of a display as in the Mindstretch product, which shows the mental recovery ($ME_{recv}$) in green during the night and the mental energy use ($ME_{load}$) in orange during the day.

Figure 26 is an example of a display as in the Mindstretch product, which shows a monthly overview of daily 24 hour measurements to show which days more mental energy was used then recovered (orange day ($ME_{load}$)) and which days more energy was recovered than used (green day ($ME_{recv}$)).

Figure 27 is a possible scheme on how objectively measured data from the invention gives alert to users and information to the experts such as general practitioner or medical specialists to give a treatment based upon measured data.

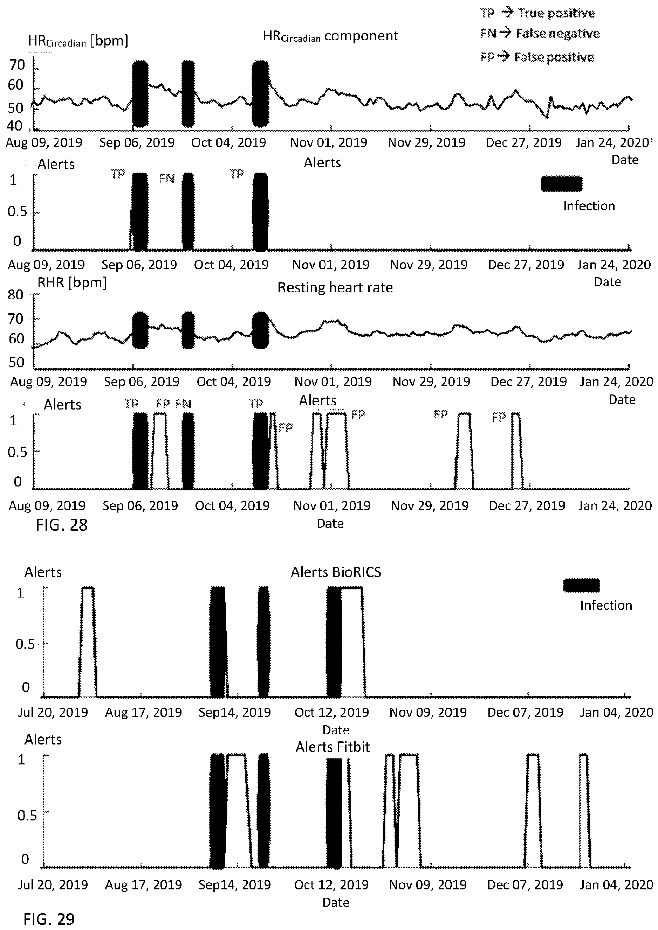

Figure 28 is an example of a comparison of results of the present invention based on evaluation of mental resilience, physical resilience and circadian basal heart rate ($HR_{circadian}$) (2 top graphs) predicting correctly infection (true positive (TP)) compared to a method based upon resting heart rate (RHR) (2 lower graphs) giving false alerts for infection (false negative (FN) and false positive (FP)).

Figure 29 is another example as in figure 28.

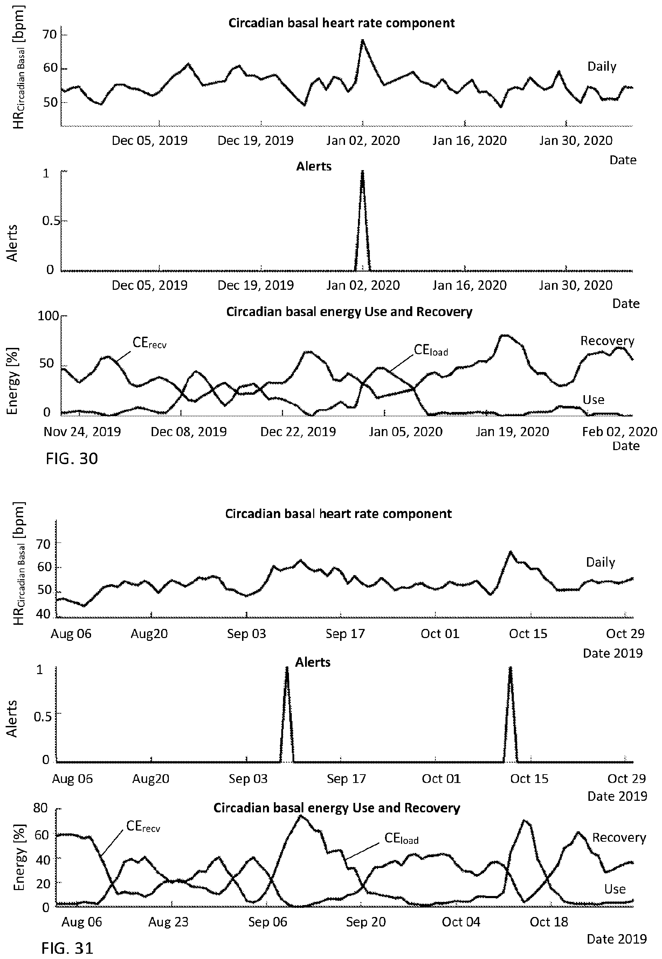

Figure 30 is an example of an infection detection wherein the detection warning is classified as a bacterial infection warning; the top graph shows the circadian basal heart rate ($HR_{circadian}$) as a daily average; the middle graph shows the alert for infection (detection warning); the lower graph shows the circadian basal energy use ($CE_{load}$) and recovery ($CE_{recv}$) as averages in a moving window of 4 days.

Figure 31 is another example of an infection detection wherein the detection warning is classified as a bacterial infection warning; the graphs are shown as in figure 30.

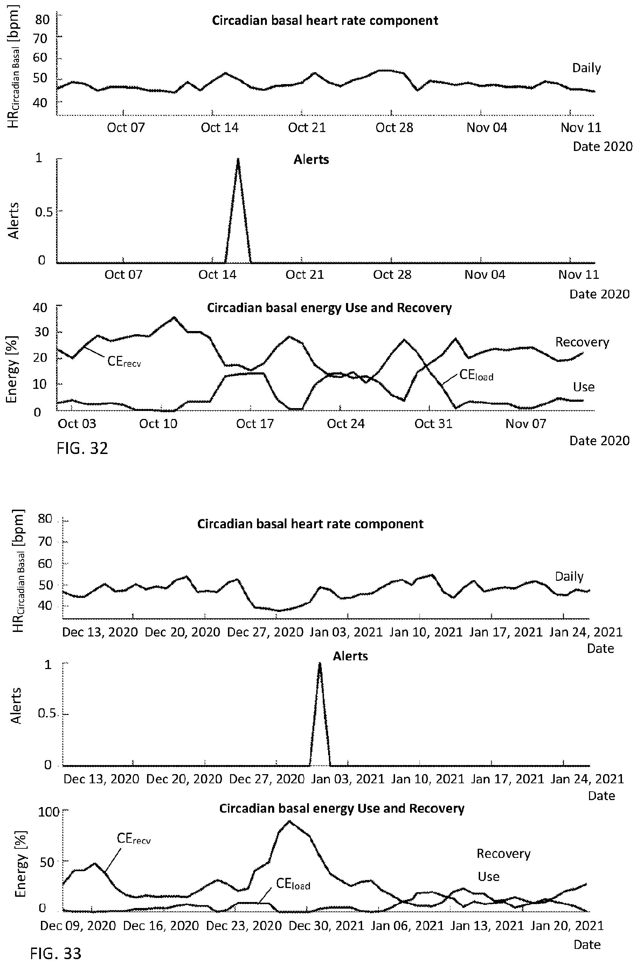

Figure 32 is example of an infection detection wherein the detection warning is classified as a viral infection warning; the graphs are shown as in figure 30.

Figure 33 is another example of an infection detection wherein the detection warning is classified as a viral infection warning; the graphs are shown as in figure 30.

## DETAILED DESCRIPTION OF THE INVENTION

The invention generally concerns a method and device to predict infection and/or inflammation by detecting vulnerability or also risk for infection and/or inflammation and use this as a basis to early and accurately detect infection and/or inflammation, based upon continuous monitoring by a detection system, preferably using wearables. The wearable delivers continuous objective physiological measurements of heart rate and physical activity, e.g. movement. These data are preferably at wearable level anonymised and encrypted and then combined with algorithms to estimate continuously (i) the use of metabolic energy versus the recovery of metabolic energy and (ii) combine this with components of heart rate to predict possible infection and/or inflammation by detecting individual risk for infection and/or inflammation and (iii) to detect infection and/or inflammation. The algorithms are adapting to each individual and continuously adapt to the individual's time-varying characteristics. The method can be applied in real-time to moving subjects in full activity.

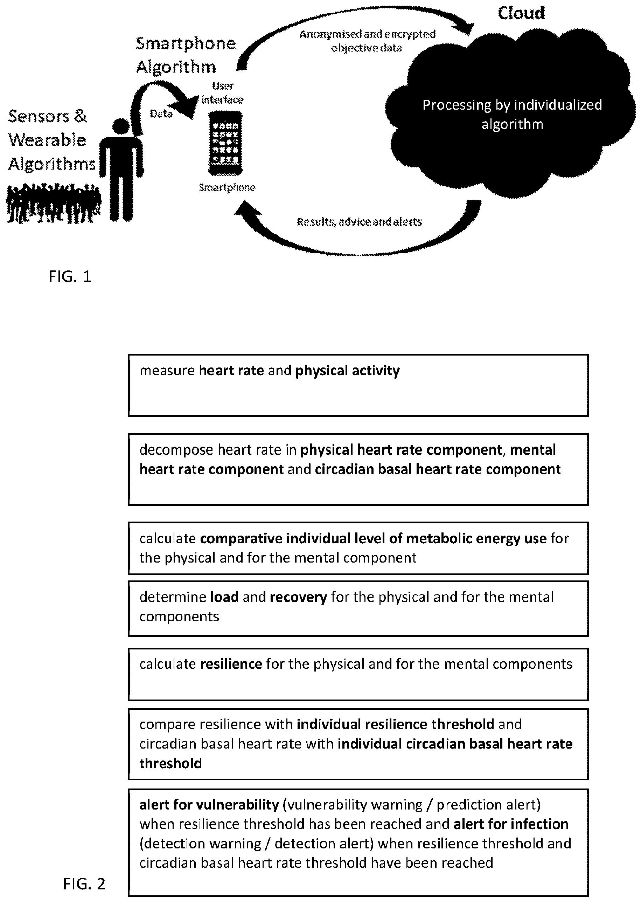

As such possibly the individual only has to wear a wearable (e.g. bracelet, watch or patch having a sensor) and/or is possibly monitored by another system (e.g. camera, sound analyser, laser system), with at least one sensor that measures continuously heart rate and physical activity, such as movement, as shown in figure 1. An application on a linked processing device, e.g. an app on a smartphone, can be used to bring preferably anonymised and encrypted data to the cloud for processing by a remote processing device. The linked and/or the remote processing device may be provided with a graphical user interface to see daily graphs of energy use versus recovery, to see monthly overviews and to get weekly advice and real-time alerts when needed. For prediction of risk for infection and/or inflammation and for detection of infection and/or inflammation, the detection system is applicable to all homoeothermic living organisms such as humans or other homoeothermic animals. The whole system can also be integrated in a bracelet, ear-tag, patch, sensor on the individual body since there is no need to be connected to data from other individuals since the system can work completely on data from only the individual. Such solution gives a watertight security respecting the GDPR rules since encrypted data and results do not leave the individual body. Hence, the detection system may comprise one or more sensors, processors and output units, and may be at least partially integrated in or linked to a smartphone and/or smartwatch.

The system can be simplified by bringing the whole algorithm within a microchip in a bracelet, patch, and sensor on or in the body. The invention can technically be realized by distributing the algorithms over the sensor (e.g. in a bracelet, a watch, a patch, etc.), the smartphone or the cloud or can be kept completely in the sensor or sensor and phone.

Figure 2 is a schematic overview visualizing the steps according to which the detection system can detect infection and/or inflammation. In the first step, heart rate and physical activity of an individual are measured such that time series of values of total heart rate and corresponding physical activity are obtained. The measurements may be done by e.g. a sensor on the body or in the environment of the individual. The measured total heart rate of the individual is then decomposed in its different components, that are at least: the circadian basal component, the physical component and the mental component. Next, a comparative individual level of metabolic energy use is calculated for the physical and for the mental component on which basis load and recovery are determined to further calculate resilience for both the physical and the mental component. The comparative individual level may be an averaged value that is individual and that is changing over time. The result of comparing the physical and mental resilience with their thresholds indicates a vulnerability for infection and/or inflammation. The vulnerability for infection and/or inflammation concerns a risk for infection and/or inflammation, and/or a prediction of possible infection and/or inflammation. Finally, both resilience and circadian basal heart rate are compared with their thresholds in order to determine whether infection and/or inflammation of the individual can be concluded. An alert for vulnerability, also called a vulnerability warning or a prediction alert, is generated when the resilience threshold has been reached and an alert for infection and/or inflammation, also called detection warning or detection alert, is generated when the resilience threshold and the circadian basal heart rate threshold have been reached.

According to a preferred embodiment the detection system comprises a sensor for measuring heart rate of the individual and a sensor for measuring physical activity, e.g. movement, of the individual for obtaining time series of heart rate and physical activity. The heart rate sensor may comprise electrodes for detecting electrical activity of the heart, an electrical sensor and/or an optical sensor. The activity sensor may comprise an accelerometer, a gyroscope and/or a GPS.

The sensor for measuring heart rate may also measure another variable that is a measurement of the metabolic energy balance of the individual and from which the heart rate may be obtained, such as respiration rate, blood oxygen level, breathing frequency, mitochondrion activity, etc. Other possible sensor techniques for obtaining heart rate are e.g. camera image analysis, sound analysis, laser technology, obtaining the heart rate signal as noise element on body movement.

The sensor for measuring physical activity may also measure another variable that measures physical performance which demands body energy of the individual and from which the physical activity may be obtained, such sensor techniques are e.g. electromyography, pedometer, camera image analysis, sound analysis, laser technology, electromagnetic field analysis etc.

Alternatively, the system may contain and use only one sensor, e.g. a motion sensor, for measuring both heart rate and movement.

According to the preferred embodiment mainly five steps of an algorithm may be identified to make the detection system operational, as shown in figure 3. The system may comprise at least one processor that is programmed according to the algorithm. A smartphone and/or a remote computer may be used as one of the processors. The algorithm may possibly run in real time or may also run after all activity and heart rate data have been collected, e.g. daily, weekly, or monthly. The whole algorithm can also be integrated in a microprocessor in a bracelet, watch, patch, ring, ear tag, etc.

In the **first step** of the algorithm of figure 3 the measured heart rate, i.e. the total heart rate, is decomposed in its different components: the circadian basal component, the physical component and the mental component.

Homoeothermic living organisms produce metabolic energy that is used for different aims with among them the most important are: keep organs functioning or the so called basal metabolism component, the immune system, control of body temperature, physical activity (e.g. running, physical performance), mental component (e.g. cognitive load, fear, stress, joy,...).

During most of the time, the metabolic energy production is done within the aerobic zone of metabolic energy production. Fresh air is breathed and in the lungs, the oxygen is transferred to the blood. The heart pumps the oxygen rich blood to the cells where the metabolic energy is produced. This means that within the aerobic zone, the amount of metabolic energy produced is proportional with the level of heart rate, such that heart rate is a measurement of the level of metabolic energy production.

In terms of energy (**EN**) and heart rate (**HR**), this can be written in their different components as follows:

$$EN_{total} = EN_{physical} + EN_{mental} + EN_{circadian} + EN_{thermal} \qquad \text{(Equation 1)}$$

$$HR_{total} = HR_{physical} + HR_{mental} + HR_{circadian} + HR_{thermal} \qquad \text{(Equation 2)}$$

wherein Equation 1 may be expressed in e.g. calories per minute or joules per minute and Equation 2 may be expressed in e.g. beats per minute; and wherein

- **EN** is metabolic energy and **HR** is heart rate;
- **$EN_{total}$** is total metabolic energy and **$HR_{total}$** is total heart rate as measured;
- **$EN_{physical}$** is the physical metabolic energy and **$HR_{physical}$** is the physical component in the total heart rate needed to deliver physical performance;
- **$EN_{mental}$** is the mental metabolic energy and **$HR_{mental}$** is the mental component in the total heart rate needed for mental activity;
- **$EN_{circadian}$** is the circadian basal metabolic energy and **$HR_{circadian}$** is the circadian basal heart rate component, which is the part of the total heart rate when the individual is awake and active at minimal level of physical performance and at relaxing mental state; this circadian basal heart rate component contains the basal heart rate (**$HR_{basal}$**) and this last one contains the heart rate for the immune system (**$HR_{immune}$**);
- **$EN_{basal}$** is the basal metabolic energy and **$HR_{basal}$** is the basal component in the total heart rate needed to provide the metabolic energy to keep the organs functioning;
- **$EN_{immune}$** is the metabolic energy for the immune system and **$HR_{immune}$** is the part of the **$HR_{basal}$** required for the immune system;
- **$EN_{thermal}$** is the thermal metabolic energy and **$HR_{thermal}$** is the part of the total heart rate required to control the body temperature.

The thermal heart rate component ($HR_{thermal}$) is relevant in specific applications where the environmental temperature is changing such as the temperature in a closed race car that rises up to 50 degree Celsius. However, for most applications according to the present invention the thermal heart rate component remains constant such that it does not need to be considered. In the few cases where this does not apply, an extra sensor for environmental temperature may be used e.g. for taking into account the part of the total heart rate required to control the body temperature.

International patent application No. WO2008/003148 (6) shows how to decompose the measured heart rate signal into at least a physical and a mental component taking into account a basal component and this in real-time on moving individuals. The method adapts to the individual and his/her time-varying characteristics. This is done by combining the measurement of heart rate in a synchronised way with the measures of body movement and with a real-time model-based algorithm that adapts to the individual continuously. In general, the decomposition may be based on differences in dynamics and responses of the heart rate components.

Physical activity of the individual requires metabolic energy and results in the corresponding physical heart rate component that can be linked to the level of activity performed and that is dependent on the individual characteristics of the individual on that moment. The measured response of the total heart rate to the physical activity allows estimating the physical component of heart rate based on synchronized measurement of the physical activity and the corresponding heart rate. The model parameters concerning the physical component may be obtained and/or evaluated when the physical heart rate component is dominantly present. The parameters are time-varying and individual such that they should be evaluated and updated over time. Mental activity also requires metabolic energy and results in the mental heart rate component that possibly is also present when physical activity is performed. The mental heart rate component may be derived from monitoring response of the individual to physical activity and the total heart rate as shown in patent application No. WO2008/003148 (6).

Each individual requires a minimal amount of metabolic energy to keep organs functioning. This so-called heart rate for the basal metabolism ($HR_{basal}$) can be measured during sleep. When heart rate is monitored continuously during the night, the basal heart rate component may be estimated for the individual with individual characteristics at that moment (e.g. age, body weight, health status, physical condition, sleep quality, etc.). The immune system also needs metabolic energy, which is reflected in the basal heart rate component. Hence, the basal heart rate component ($HR_{basal}$) includes the heart rate component of the immune system ($HR_{immune}$). The basal heart rate component ($HR_{basal}$), is further part of the circadian basal heart rate component ($HR_{circadian}$). The last one being the variable component of the total heart rate that is present when the individual is awake with minimal physical activity and relaxing mental state. The basal heart rate component ($HR_{basal}$) fluctuates around a constant value during a time window of one day and therefore is according to the state of the art so far reduced to a constant value that is mostly called the "Resting Heart Rate" (RHR). The circadian basal heart rate component ($HR_{circadian}$) is however varying over a 24-hour period with the basal heart rate ($HR_{basal}$) as its minimal value. Consequently, since the basal heart rate component ($HR_{basal}$) contains the heart rate component of the immune system ($HR_{immune}$), the latter also affects the circadian basal heart rate component ($HR_{circadian}$).

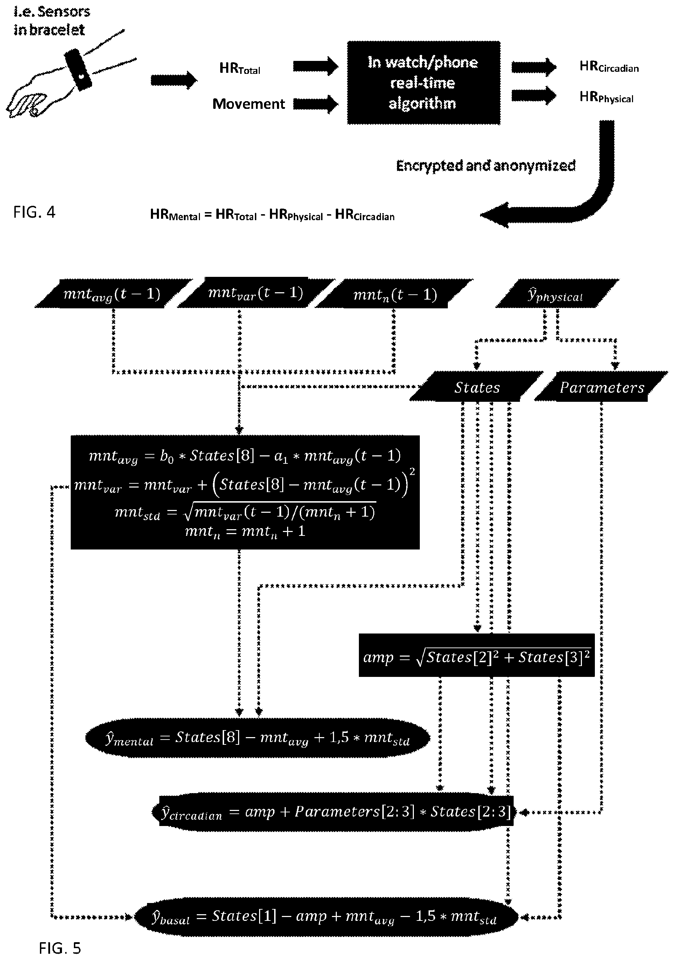

According to the current invention the circadian basal heart rate component ($HR_{circadian}$) may further also be monitored and calculated in real-time when mental and physical activity are present, e.g. when the individual is awake and/or is moving in full activity. A general scheme that may be applied is represented in figure 4.

Continuously collecting data on individuals when sleeping, moving and/or not moving as in the present invention, allows measuring the total heart rate and decomposing it in the different components. Figure 5 shows a possible scheme of an algorithm for decomposing the total heart and calculating the circadian basal heart rate component ($HR_{circadian}$) according to the invention based on continuously collected data of total heart rate and physical activity. The algorithm of figure 5, as example, makes use of a state-space representation modelling technique for decomposing the total heart rate in its different components for physical activity ($HR_{physical}$), mental activity ($HR_{mental}$), basal metabolism ($HR_{basal}$) and circadian basal metabolism ($HR_{circadian}$).

In the scheme

$\hat{y}_{physical}$ is the physical heart rate component estimated using heart rate and activity data directly from the wearable;

$mnt_{avg}$ is the average of the mental component calculated using data from the last 31 days once enough data is available. Before having this amount of data, just using all data available;

$mnt_{var}$ is the variance of the mental component calculated using data from the last 31 days once enough data is available. Before having this amount of data, just using all data available;

$mnt_n$ is the amount of mental component samples used to estimate the statistical metrics;

$mnt_{std}$ is the standard deviation of the mental component calculated using data from the last 31 days once enough data is available. Before having this amount of data, just using all data available;

**States** is the matrix representing the heart rate components values in the state-space representation;

**Parameters** is the matrix representing the parameters values in the state-space representation;

**States [i]** is the state-space representation value of the heart rate component I;

**Parameters [i]** is the state-space representation value of the parameter I;

$a_1$ is the denominator parameter value for a first order transfer function model;

$b_0$ is the numerator parameter value for a first order transfer function model;

**amp** is the amplitude of the circadian basal component, which is the maximal difference in values during the oscillating value of the circadian basal component;

$\hat{y}_{basal}$ is the basal heart rate component value;

$\hat{y}_{circadian}$ is the circadian basal heart rate component value;

$\hat{y}_{mental}$ is the mental heart rate component value.

Besides the state-space representation modelling technique, other suitable modelling techniques are known as such. Examples are data-based mechanistic input-out modelling, deconvolution methods, time series analysis, singular value decomposition, principal component analysis, neural networks, etc.

The resulting decomposed components $HR_{physical}$, $HR_{mental}$ and $HR_{circadian}$ are shown in figure 6 for a period of about 2 weeks.

Figure 7 shows that this approach gives significant differences from what is done so far when using a so-called Resting Heart Rate according to the state of the art as a daily constant value. Figure 7 shows for an individual examples of the difference over 24 hours (Figure 7) and over 2 weeks (Figure 8) between the Resting Heart Rate, as used so far according to the state of the art, and the circadian basal heart rate component, as resulting from the present invention. It can be noticed that the quantitative difference between the so-called Resting Heart Rate and the $HR_{circadian}$ can be 14 bpm! The figure also shows that according to the present invention $HR_{basal}$ and $HR_{circadian}$ are not constant values as considered in other methods according to the state of the art. The amplitude of $HR_{circadian}$ in this example reaches 14 bpm (Figure 7).

Figure 9 shows for an individual the difference between the total, the physical and the mental heart rate during an active day. The figure shows for an individual that the mental heart rate component and the physical heart rate component may significantly vary during and active day. Figure 10 shows the same values for a longer measuring period of about 3 months.

Figure 10 shows the decomposition of heart rate of an individual in its different components over a period of 3 months and figure 11 shows the corresponding activity during this period. Figure 12 shows the decomposition of heart rate of an individual in its different components ($HR_{mental}$, $HR_{physical}$ and $HR_{circadian}$) over a period of 24 hours and figure 13 shows the corresponding activity during this period.

In the **second step** of the algorithm of figure 3, use of metabolic energy or energy expenditure is compared with recovery of metabolic energy for both the physical component and the mental component.

All activities of homoeothermic living organisms use metabolic energy, which is also referred to as metabolic energy load or energy expenditure. Different activities may be sleeping, moving or physical performance, mental load like fear, anger, cognitive load, happiness, etc. The activities require that metabolic energy be created by the organism during action. Furthermore, at some point the organism needs recovery of the metabolic energy used.

The algorithm of the detection system calculates recovery of the individual. This is preferably done in a fully automated way from data of a wearable device collecting continuously heart rate and physical activity, e.g. movement.

The continuously estimated mental heart rate component ($HR_{mental}$) and physical heart rate component ($HR_{physical}$) are measures for, respectively, metabolic energy use of the mental energy component and metabolic energy use of the physical energy component.

From the estimated mental heart rate component ($HR_{mental}$) and the estimated physical heart rate component ($HR_{physical}$), average levels of each of these components are calculated. The averages of both components are individually different and are referred to as comparative individual levels of metabolic energy use. These are measures for the individual average energy use with respect to the mental and the physical components. Preferably, the algorithm calculates it over about one month, i.e. the preceding month. When the current level of energy use of an energy component, is lower than its average level, it is considered that the individual is recovering for this energy component.

As such when e.g. the current level of mental energy use of an individual, is lower than the average level of mental energy of this individual, it is considered that the individual is mentally recovering, which may be since the individual is not mentally engaged at high level and is mentally relaxing.

By comparing the levels of mental energy use, as connected to levels of activity, it is possible to see whether the individual is mentally recovering during an activity or burning more mental energy.

Figure 14 and 15 show a possible scheme of further algorithms to calculate load and recovery for, respectively, the mental metabolic energy and the physical metabolic energy.

In the scheme further:

> $y_{non\_physical}$ is the sum of the non-physical components;
> *sr* is the array to store the mental load/recovery value;
> *lr* is the array to store the physical load/recovery value;
> $phys_{std}$ is the standard deviation of the physical component calculated using data from the last 31 days once enough data is available. Before having this amount of data, just using all data available;
> $phys_{med}$ is the median of the physical component calculated using data from the last 31 days once enough data is available. Before having this amount of data, just using all data available;
> *Ment_Load* is the mental energy load;
> *Ment_Recv* is the mental energy recovery;
> *Phys_Load* is the physical energy load;
> *Phys_Recv* is the physical energy recovery.

Figure 16 shows the resulting calculated load, i.e. energy use, and recovery for the mental and the physical components.

In the **fourth step** of the algorithm of figure 3, the physical resilience and the mental resilience are calculated based on the energy load and the energy recovery for, respectively, the physical and the mental component.

In healthy individuals a balance between use of metabolic energy and recovery of energy is present. The use of metabolic energy is also called energy load or energy expenditure. When for a longer period more energy is used than what the individual recovers from food, rest, sleep and for mental recovery also from mentally relaxing events, some of the components in the metabolic energy balance are going in shortage of energy. During high mental stress peaks, the immune system is depressed to save energy for the fly or fight reaction. When there is a chronic high use of mental energy due to mental stress, then there are effects on the bioenergetic system due to a lack of homeostatic balance. A shortage of recovery of metabolic energy due to high mental energy expenditure or due to physical energy expenditure, or both, increases the risk for the individual going into a risk for infection and/or inflammation.

According to the invention the resilience is calculated based upon the ratio between recovery -i.e. production of metabolic energy- versus load -i.e. use of metabolic energy. The ratio is different for each individual and even individually time-varying. When the ratio results in a high availability of metabolic energy, it is assumed that the individual has a high resilience. A low resilience shows the opposite. Since the ratio between load and recovery is individually different and time-varying it, preferably, must be calculated continuously. Hence, according to the preferred embodiment the resilience is calculated continuously over time.

As stated higher the different steps to switch on the immune system and make it work take metabolic energy. This means that, depending on the energy status of the body of the individual, the risk for infection and/or inflammation is higher when for a too long time, there is a lack of recovery and energy production is low compared to energy use. A low resilience gives a higher risk for infection and/or inflammation because the immune system soon is in lack of enough energy when switched on.

A most important concept is that the estimation of resilience allows to detect vulnerability for infection and/or inflammation or high risk for infection and/or inflammation due to low resilience and this allows (i) prediction of infection and/or inflammation with early detection of infection and/or inflammation and (ii) more accurate detection of infection and/or inflammation in terms of true and false positives and negatives.

To realise this scheme, given in figure 17, a possible scheme of a further algorithm to calculate mental and physical resilience is shown in figure 18.

In the scheme further

$ME\_Load$ or $ME\_Use$ is the daily mental energy load;
$ME\_Recv$ is the daily mental energy recovery;
$MP\_Load$ or $MP\_Use$ is the daily physical energy load;
$MP\_Recv$ is the daily physical energy recovery;
$Res_{ment}$ is the mental resilience;
$Res_{phys}$ is the physical resilience;
$Res_{ment}(t)$ is the mental resilience at time t;
$Res_{phys}(t)$ is the physical resilience at time **t**.

As such, according to the preferred embodiment, the algorithm may obtain the physical and the mental resilience as follows.

The physical resilience for a day may be estimated as the ratio between the area under curve made by the values of the physical component during the time awake (for example between 07:01 and 23:59) weighed by the maximal physical effort and the area under the recovery estimation during the sleep (for example between 00:00 and 07:00) weighed by the maximal recovery.

The mental resilience for a day may be estimated as the ratio between the total sum of the mental energy use over the total sum of the mental energy recovery.

A possible scheme of a further algorithm to evaluate mental and physical resilience to predict vulnerability for infection and/or inflammation is shown in figure 19.

In the scheme further

$Prediction_{ment}$ is the prediction raised by mental resilience;
$Prediction_{phys}$ is the prediction raised by physical resilience.

The shortage of energy for the immune system can come from a lack of resilience in the physical component or from a lack of resilience in the mental component or in both.

An alert for vulnerability to infection and/or inflammation, or also called herein a prediction alert or a vulnerability warning, is raised when the mental resilience and/or the physical resilience is smaller than or equal to a threshold of one.

Another possible scheme of a further algorithm to evaluate mental and physical resilience to predict vulnerability for infection and/or inflammation is shown in figure 20.

The alert for vulnerability to infection and/or inflammation is raised when the following two criteria are met for the mental resilience and/or for the physical resilience.

One criterion is based on a short-term comparison of resilience values obtained over time. The previous day resilience value is subtracted from the current day resilience value. The criteria are met when the difference is equal or minor than a threshold. The threshold may be a value of for instance -5.0 for physical resilience and -5.0 for mental resilience.

The other criterion is based on a long-term comparison of resilience values obtained over time. The daily resilience value of the previous week is subtracted from the current day resilience value. The daily resilience value of the previous week is hereby an average preceding resilience of days of the preceding week. It corresponds to an average value of daily resilience values of the preceding week. The average preceding resilience may also be calculated over shorter or longer periods of e.g. only three days, one month or several months. The criteria are met when the difference is equal or minor than a threshold. The threshold may be a value of for instance -10.0 for physical resilience and - 15.0 for mental resilience.

Hence, if both the short-term and the long-term criteria are met, then the system detects that the individual is vulnerable to infection and/or inflammation and a higher risk of infection and/or inflammation may be predicted such that an alert of vulnerability for infection and/or inflammation is raised. The result of this is shown by an example in figure 21.

In the **third step** as shown in figure 3, the circadian basal component of the heart rate is involved to check whether the individual threshold of it is surpassed. This individual threshold may be estimated by statistical metrics of historical data of the individual.

The individual threshold may possibly be calculated as a comparative individual level of metabolic energy use for the circadian basal metabolism based on preceding time series of the circadian basal heart rate heart rate. It is preferably calculated as an average value of the preceding values of circadian basal heart rate heart rate. The preceding time series may correspond to a period of about one month. As such the individual threshold may change over time.

Furthermore, also dynamics of the circadian basal heart rate component may possibly be analysed by comparing the current circadian basal heart rate component with a preceding circadian basal heart rate component of a preceding timeframe or period and then determining whether a circadian basal heart rate threshold has been reached.

Possibly a fast trend and a slow trend of the circadian basal heart rate component may be evaluated. The current circadian basal heart rate component may be compared with the immediately preceding circadian basal heart rate component of an immediately preceding timeframe, corresponding to a change over a short period of 1 to 2 days, and a threshold indicating a fast change. The current circadian basal heart rate component may further be compared with an average preceding circadian basal heart rate component of a number of preceding timeframes, corresponding to a change over a long period of 10 to 40 days, and a threshold indicating a slow change. When the fast threshold and the slow threshold have been reached an alert may be generated.

In the **fifth step** as shown in figure 3, the prediction of vulnerability based on evaluation of the physical and mental resilience is combined with evaluation of the circadian basal component of heart rate.

When the alert for high vulnerability or risk for infection and/or inflammation is given and at the same time the individual threshold for circadian basal component is reached then this results in the conclusion that an early stage of infection and/or inflammation is present and a detection alert is generated as shown in figures 22 and 23.

The resulting alert may be generated on a display. The display may comprise the display of a smartphone and/or a remote computer monitor.

The commercialised BioRICS' Mindstretch product shows an example of a smartphone and/or a smartwatch used as sensor, processor and with a display as output unit wherein mental recovery during the night is shown in green and mental energy use during the day is shown in orange as represented in figure 24. The figure shows as an example a 46 % mental recovery over 24 hours. This 46 % of mental recovery results in a day where more mental energy was used than recovered and consequently in Mindstretch that leads to an orange day as shown in Figure 25.

Mindstretch shows only mental energy use and recovery, but no physical energy use neither recovery nor mental/physical resilience since Mindstretch is only focusing on the mental component. For athletes it also gives the total heart rate during physical activity but no further results (Figure 25).

The Mindstretch product in figures 24 and 25 shows for each day of the month which days the individual recovers more than (green day) or less than (orange day) the amount of burned energy. A green day is a day where the body has produced more recovery of mental energy than used during these 24 hours. An orange day is a day where the user has used more mental energy than the body has recovered during those 24 hours.

Figure 26 shows a monthly overview wherein orange days indicate that more metabolic mental energy is used than recovered and green days show that more energy was recovered than used.

There is no problem when an individual has several days where more mental energy is used than the amount that has been recovered in those 24 hours, but sooner or later, the burned mental energy must be recovered. This used mental energy is not only due to negative stress but also to positive mental events such as cognitive load, mental engagement during barbecue with friends etc.

The Mindstretch product could be adapted to operate as a detection system according to the present invention. Evaluating mental and physical resilience may possibly be done by analysing the ratio between green and yellow days or even more accurate by calculating the surface or the mathematical integral under the measured curves of mental and physical energy use and calculate the ratio between the total green values and orange values over a longer period.

EXAM PLES

Figures 22 and 23 show examples of a monitored individual going into being sick. Heart rate and movement are measured and monitored continuously. Figure 22 shows clearly that the different heart rate components vary when the subject gets an infection. The detection system, when using the algorithm to analyse the metabolic energy use, the resilience, the circadian basal heart rate component, detects and alerts three different events, namely hiking above 3000 m that takes a lot of metabolic energy (1), the presence of a flu, i.e. a viral infection (2) and a bacterial infection (3). Extreme or unusual activities may also affect the detection system and show an increase in vulnerability and/or a change in circadian basal metabolism. To easily detect and classify extreme or unusual activities such as hiking above 3000 m, the detection system may be combined with e.g. a GPS in a smartphone.

To demonstrate the difference between the method of the detection system of the present invention with methods of detection systems of the state of the art that are mainly based upon using the resting heart rate (RHR), both methods have been applied on the same data set of subjects getting infected. Figures 28 and 29 show the differences with correct early warning by prediction for possible infection and/or inflammation and correct detection of infection versus many false positives when the resting heart rate is used to detect infections.

Both methods were compared on a data set from 7 different subjects followed over a period of 8 months with in total 13 infections, from which one Corona infection. Running both methods over all these data, it can be concluded that the accuracy of the present invention is significantly better. From sensitivity, specificity and resulting accuracy calculated for the detection of infections for both methods, as presented in table 1, it can be concluded that relying upon evaluation of the evolution of physical resilience, mental resilience and circadian basal heart rate compared to relying only on the evolution of resting heart rate gives significantly better results.

Table 1: the method of the present invention compared with a method using the resting heart rate applied on 13 infections from 7 subjects over a period of 8 months.

|  | Sensitivity | Specificity | Accuracy |
|---|---|---|---|
| Method according to the present invention | 90 % | 84 % | 84 % |
| Method based upon resting heart rate | 64 % | 47 % | 42 % |

Naturally, the invention is not restricted to the method and device according to the invention as described above. Thus, the detection system may be part of a closed loop system for preventive personal health management and monitoring based treatment as shown in figure 27. Furthermore, the detection system may also be part of a global monitoring system for predicting and detection of infectious disease outbreak in populations.

Further, it has been found that infections caused by viruses and infections caused by bacteria do have a different effect on the metabolic energy use for the circadian basal metabolism. Moreover, a difference in the effect on the circadian basal metabolism is found to be present before the detection warning, i.e. the infection warning. Hence, the invention further allows to distinguish viral and bacterial infections.

Based on preceding time series of the circadian basal heart rate heart rate component comparative individual levels of metabolic energy use for the circadian basal metabolism are calculated. Further, current individual levels of metabolic energy use for the circadian basal metabolism are calculated based on the current circadian basal heart rate component.

Recovery is determined when the current individual level of metabolic energy use is lower than the comparative individual level of metabolic energy use for the circadian basal metabolism. Load is determined when the current individual level of metabolic energy use is higher than the comparative individual level of metabolic energy use for the circadian basal metabolism.

The infection warning is then classified as a bacterial infection warning or as a viral infection warning. The infection warning is classified as a bacterial infection warning when the metabolic energy use for the circadian basal metabolism increases between 10 and 25 days before the infection warning and at the moment of the infection warning. The infection warning is classified as a viral infection warning when recovery for the circadian basal metabolism increases between about 5 to 6 days before the infection warning and the metabolic energy use for the circadian basal metabolism increases about 10 day after the infection warning. Possibly, the infection warning may also be classified as a viral infection warning when it is not classified as a bacterial infection warning.

Figure 30 shows an example of a bacterial infection detection. There is an increase in circadian basal energy use versus recovery, which peaks at day 23 and day 15 before the bacterial infection detection, i.e. the detection warning that is classified as a bacterial infection warning. There is another increase in circadian basal energy use right at detection until some days after (3 days in this case).

Figure 31 shows another example of a bacterial infection detection. There is an increase in circadian basal energy use versus recovery which peaks at 25 and 10 days before the bacterial infection detection, i.e. the detection warning that is classified as a bacterial infection warning. There is another increase in circadian basal energy use right at both detections which lasts until some days after, i.e. 10 and 5 days, respectively.

Figure 32 shows an example of a viral infection detection. There is an increase in circadian basal energy recovery ranging from 5 to 2 days prior to viral infection detection, i.e. the detection warning that is classified as a viral infection warning. There is an increase in circadian basal energy use from day 10 to 13 after infection detection.

Figure 33 shows another example of a viral infection detection. There is an increase in circadian basal energy recovery ranging until 6 days prior to viral infection detection, i.e. the detection warning that is classified as a viral infection warning. There is an increase in circadian basal energy use from day 10 to 13 after infection detection.

In the presented examples for a bacterial infection detection there is an increase in circadian basal energy use between 5 and 30 days, in particular between 10 and 25 days, before the infection and an increase in circadian basal energy use at the moment of infection detection. In the examples for viral Infection detection there is an increase in circadian basal energy recovery between 2 and 9 days, in particular between 5 and 6 days, before the infection and an increase in circadian basal energy use between 5 and 15 days, in particular around 10 days, after infection detection. For both types of infection it is also possible to detect the end of symptoms of the infection when circadian basal energy recovery goes again higher than the energy use.

REFERENCES

(1) Hans Selye 70 years later: steroids, stress ulcers and H. Pylori. Azabo S., 2014. Clin. Neuroscience, 69, 85-93.
(2) "Stress is 80 years old: from Hans Selye original paper in 1936 to recent advances in GI Ulceration. Szabo S., Yoshida M., Filakovszky J and Juhasz G., 2014. Current Pharmaceutical Design, 2017, 23, 4029-4041.
(3) Automated real-time stress monitoring of police horses using wearable technology. Norton, T; Piette, D. ; Exadaktylos, V. ; Berckmans, D. 2018. APPLIED ANIMAL BEHAVIOUR SCIENCE, 198, 67-74, DOI: 10.1016/j.applanim.2017.09.009
(4) www.biorics.com
(5) Dynamic modelling of the cardiovascular response to a combined physical and mental task to identify biomarkers for psychopathology. Piette D., Norton T., Vrieze E., Berckmans D., Aerts J.M., Exadaktylos V., Bogaerts K., Hompes T., Claes S., to be submitted to Psychological Medicine.
(6) Real-time monitoring and control of physical and arousal status of individual organisms PCT/BE2007/000075 (WO2008/003148)
(7) Investigation of an estimate of daily resting heart rate using a consumer wearable device Russell A., Heneghan C., and Venkatraman S. 2019. medRxiv, published on-line Oct 18. Pp 1-10, https://doi.org/10.1101/19008771doi:
(8) Harnessing wearable device data to improve state-level real-time surveillance of influenza-like illness in the USA: a population-based study. 2020. Jennifer M Radin, Nathan E Wineinger, Eric J. Topol, Steven R Steinhubl. The Lancet.com/digital-health, e85-393.
(9) Brage S, Brage N, Franks PW, Ekelund U, Wareham NJ (2005). Reliability and validity of the combined heart rate and movement sensor Actiheart. Eur J Clin Nutr 59, 561-570
(10) Segerstrom, S.C., 2010. Resources, Stress, and Immunity: An Ecological Perspective on Human Psychoneuroimmunology. ANNALS OF BEHAVIORAL MEDICINE, 40, 1, 114-125. DOI: 10.1007/s12160-010-9195-3
(11) Segerstrom, S.C., Miller, G.E., 2004. Psychological stress and the human immune system: A meta-analytic study of 30 years of inquiry. PSYCHOLOGICAL BULLETIN, 130, 4, 601-630. DOI: 10.1037/0033-2909.130.4.601
(12) Selye H., 1975, The stress of life. Mc Graw Hill, New York.
(13) Fowler G.A., 2020. Wearable tech can spot coronavirus symptoms before you even realize you're sick. Technology columnist May 28, 202 The Washington Post.

**Claims**

1. Detection system for detecting vulnerability for infection and/or inflammation and for detecting infection and/or inflammation of a homeothermic living organism, the system comprising:

   - at least one sensor to measure and monitor as function of time heart rate and physical activity of the living organism for obtaining time series of heart rate and physical activity;
   - at least one processor programmed to

     ■ decompose the heart rate in at least time series of a **physical heart rate component** due to physical activity ($HR_{physical}$), time series of a **mental heart rate component** due to mental activity ($HR_{mental}$) and time series of a **circadian basal heart rate component** due to circadian basal metabolism ($HR_{circadian}$);
     ■ calculate **comparative individual levels of metabolic energy use** for the physical activity and the mental activity based on preceding time series of, respectively, at least the physical and at least the mental heart rate components;
     ■ calculate **current individual levels of metabolic energy use** for the physical activity and the mental activity based on current heart rate components;
     ■ determine **recovery** when the current individual level of metabolic energy use is lower than the comparative individual level of metabolic energy use for the physical activity and the mental activity;
     ■ determine **load** when the current individual level of metabolic energy use is higher than the comparative individual level of metabolic energy use for the physical activity and the mental activity;

- calculate **resilience** based on the ratio between the recovery and the load for the physical activity and the mental activity;
- compare current resilience with at least one **resilience threshold** for the physical activity and the mental activity and determine whether the at least one resilience threshold has been reached for the physical activity and/or the mental activity;
- compare current circadian basal heart rate component with at least one **circadian basal heart rate threshold** and determine whether the at least one circadian basal heart rate threshold has been reached;

- an output unit configured to generate at least one result that comprises a detection warning when the processor determines that

- the at least one resilience threshold has been reached and the at least one circadian basal heart rate threshold has been reached.

2. Detection system according to claim 1, wherein the at least one result comprises a vulnerability warning when the processor determines that the at least one resilience threshold has been reached for the physical activity and/or the mental activity.

3. Detection system according to claim 1 or 2, wherein the comparative individual levels of metabolic energy use are levels obtained from preceding time series of the heart rate components, preferably over a previous time window of about 2 to 60 days, more preferably a period of 10 to 40 days, in particular a period of about one month.

4. Detection system according to any of the claims 1 to 3, wherein comparing current resilience with at least one resilience threshold for the physical activity and the mental activity comprise comparing current resilience with at least one preceding resilience and determining whether the at least one resilience threshold has been reached for the physical activity and the mental activity.

5. Detection system according to claim 4, whereby comparing current resilience with preceding resilience comprise subtracting at least one preceding resilience from the current resilience and determining that the at least one resilience threshold has been reached when the at least one preceding resilience subtracted from the current resilience is lower than the at least one resilience threshold.

6. Detection system according to claim 4 or 5, whereby the at least one resilience threshold comprise a **long term resilience threshold,** whereby comparing current resilience with at least one preceding resilience and determining whether the at least one resilience threshold has been reached comprise comparing the current resilience with an **average preceding resilience** of a number of preceding timeframes and determining whether the long term resilience threshold has been reached, and wherein the output unit is further configured to generate the result when the processor determines that the long term resilience threshold has been reached.

7. Detection system according to claim 6, whereby the at least one resilience threshold further comprise a **short term resilience threshold**, whereby comparing current resilience with preceding resilience and determining whether the at least one resilience threshold has been reached further comprise comparing the current resilience with the **immediately preceding resilience** of an immediately preceding timeframe and determining whether the short term resilience threshold has been reached and wherein the output unit is further configured to generate the result when the processor determines that both the short term resilience threshold and the long term resilience threshold have been reached.

8. Detection system according to any of the claims 1 to 7, whereby the at least one processor is further programmed to calculate comparative individual levels of metabolic energy use for the circadian basal metabolism based on preceding time series of the circadian basal heart rate heart rate for use as the at least one circadian basal heart rate threshold.

9. Detection system according to any of the claims 1 to 8, whereby the at least one processor is further programmed to analyse dynamics of the circadian basal heart rate component by comparing the current circadian basal heart rate component with at least one preceding circadian basal heart rate component of at least one preceding timeframe and determining whether the at least one circadian basal heart rate threshold has been reached.

10. Detection system according to claim 9, whereby

- the at least one processor is further programmed to analyse dynamics of the circadian basal heart rate component by

  - comparing the current circadian basal heart rate component with the **immediately preceding circadian basal heart rate component** of an immediately preceding timeframe and a **fast circadian basal heart rate threshold** of the at least one circadian basal heart rate threshold; and
  - comparing the current circadian basal heart rate component with an **average preceding circadian basal heart rate component** of a number of preceding timeframes and a **slow circadian basal heart rate threshold** of the at least one circadian basal heart rate threshold;

- the output unit is further configured to generate the result when the processor also determines that

  - the fast circadian basal heart rate threshold and the slow circadian basal heart rate threshold have been reached.

11. Detection system according to claim 9 or 10, whereby the at least one processor is further programmed to analyse dynamics of the circadian basal heart rate component by comparing with the at least one threshold

   - a fast trend of the circadian basal heart rate component, corresponding to a change over a short period of 1 to 9 days, preferably 1 to 7 days, in particular, 1 to 2 days, and
   - a slow trend of the circadian basal heart rate component, corresponding to a change over a long period of 10 to 40 days, preferably 10 to 30 days, in particular 20 to 28 days.

12. Detection system according to any of the claims 1 to 11, whereby the at least one sensor comprises an accelerometer, a gyroscope, a motion sensor, a GPS, a camera, an electrical sensor, an optical sensor, an electrocardiogram device, a heart sound sensor, a laser device, a magnetic field sensor, a pedometer and/or a sound analyser; and/or whereby the detection system is at least partially integrated in a wearable device such as a smart watch, smart phone, breast band, bracelet, patch and/or sticker.

13. Detection system according to any of the claims 1 to 12, whereby resilience is calculated in a timeframe of at least one day and whereby the current resilience is the resilience of at least the current day.

14. Detection system according to any of the claims 1 to 13, whereby the timeframe corresponds to a period of at least one day and the number of preceding timeframes corresponds to a total period of 3 to 60 days, in particular a period of about one week or about one month.

15. Detection system according to any of the claims 1 to 14, whereby the at least one processor is further programmed to

   - calculate comparative individual levels of metabolic energy use for the circadian basal metabolism based on preceding time series of the circadian basal heart rate heart rate component;
   - calculate current individual levels of metabolic energy use for the circadian basal metabolism based on the current circadian basal heart rate component;
   - determine recovery when the current individual level of metabolic energy use is lower than the comparative individual level of metabolic energy use for the circadian basal metabolism; and
   - determine load when the current individual level of metabolic energy use is higher than the comparative individual level of metabolic energy use for the circadian basal metabolism;
   for classifying the detection warning as a bacterial infection warning or as a viral infection warning, whereby the at least one processor is still further programmed to
   - classify the detection warning as a bacterial infection warning when the processor determines that the metabolic energy use for the circadian basal metabolism increases between 5 and 30 days, preferably between 10 and 25 days, before the detection warning and at the moment of the detection warning; and/or
   - classify the detection warning as a viral infection warning when the processor determines that the recovery for the circadian basal metabolism increases between 2 and 9 days, preferably between 5 to 6 days, before the detection warning and the metabolic energy use for the circadian basal metabolism increases between 5 and 15 days, preferably about 10 day, after the detection warning;

   whereby the output unit is further configured to generate the at least one result that comprises a viral infection warning and/or a bacterial infection warning.

16. A computer readable medium storing a computer program and instructions for performing a method for predicting vulnerability and detecting infection and/or inflammation of a homeothermic living organism, the method comprising:

- measuring and monitoring, using at least one sensor, as function of time heart rate and physical activity of the living organism for obtaining time series of heart rate and physical activity;
- decomposing, using at least one processor, the heart rate in at least time series of a physical heart rate component due to physical activity ($HR_{physical}$), time series of a mental heart rate component due to mental activity ($HR_{mental}$) and time series of a circadian basal heart rate component due to basal metabolism ($HR_{circadian}$);
- calculating, using the at least one processor, comparative individual levels of metabolic energy use for the physical activity and the mental activity based on preceding time series of the heart rate components for the physical activity and, respectively, the mental activity;
- calculating, using the at least one processor, current individual levels of metabolic energy use for the physical activity and the mental activity based on current heart rate components;
- determining, using the at least one processor, recovery for the physical activity and the mental activity when the current individual level of metabolic energy use is lower than the comparative individual level of metabolic energy use;
- determining, using the at least one processor, load for the physical activity and the mental activity when the current individual level of metabolic energy use is higher than the comparative individual level of metabolic energy use;
- calculating, using the at least one processor, resilience for the physical activity and the mental activity based on the ratio between the recovery and the load;
- comparing, using the at least one processor, current resilience for the physical activity and the mental activity with at least one resilience threshold;
- comparing, using the at least one processor, current circadian basal heart rate component with at least one circadian basal heart rate threshold;
- generating a result using an output unit when the processor determines that the at least one resilience threshold and the at least one circadian basal heart rate threshold have been reached.

## Patentansprüche

1. Detektionssystem zum Erkennen der Anfälligkeit für Infektionen und/oder Entzündungen und zum Erkennen von Infektionen und/oder Entzündungen eines homöothermen lebenden Organismus, wobei das System umfasst:

- mindestens einen Sensor zum Messen und Überwachen der Herzfrequenz und der körperlichen Aktivität des lebenden Organismus in Abhängigkeit von der Zeit, um Zeitreihen der Herzfrequenz und der körperlichen Aktivität zu erhalten;
- mindestens einen Prozessor, der programmiert ist, um

- die Herzfrequenz in mindestens Zeitreihen einer **körperlichen Herzfrequenzkomponente** aufgrund von körperlicher Aktivität ($HR_{physical}$), Zeitreihen einer **geistigen Herzfrequenzkomponente** aufgrund geistiger Aktivität ($HR_{mental}$) und Zeitreihen einer **zirkadianen Basalherzfrequenzkomponente** aufgrund des circadianen Basalmetabolismus ($HR_{circadian}$) zu zerlegen;
- **vergleichende individuelle Niveaus des metabolischen Energieverbrauchs** für die körperliche Aktivität und die geistige Aktivität auf der Grundlage vorhergehender Zeitreihen von jeweils zumindest der körperlichen und zumindest der geistigen Herzfrequenzkomponenten zu berechnen;
- die **aktuellen individuellen Niveaus des metabolischen Energieverbrauchs** für die körperliche Aktivität und die geistige Aktivität auf der Grundlage der aktuellen Herzfrequenzkomponenten zu berechnen;
- die **Erholung** zu bestimmen, wenn das aktuelle individuelle Niveau des metabolischen Energieverbrauchs niedriger ist als das vergleichende individuelle Niveau des metabolischen Energieverbrauchs für die körperliche Aktivität und die geistige Aktivität;
- die **Belastung** zu bestimmen, wenn das aktuelle individuelle Niveau des metabolischen Energieverbrauchs höher ist als das vergleichende individuelle Niveau des metabolischen Energieverbrauchs für die körperliche Aktivität und die geistige Aktivität;
- die **Belastbarkeit** auf der Grundlage des Verhältnisses zwischen der Erholung und der Belastung für die körperliche Aktivität und die geistige Aktivität zu berechnen;
- die aktuelle Belastbarkeit mit mindestens einem **Belastbarkeitsschwellenwert** für die körperliche Aktivität und die geistige Aktivität zu vergleichen und zu bestimmen, ob der mindestens eine Belastbarkeitsschwellenwert für die körperliche Aktivität und/oder die geistige Aktivität erreicht wurde;
- die aktuelle zirkadiane Basalherzfrequenzkomponente mit mindestens einem **Schwellenwert für die zirkadiane Basalherzfrequenz** zu vergleichen und zu bestimmen, ob der mindestens eine Schwellenwert für die zirkadiane Basalherzfrequenz erreicht wurde;

- eine Ausgabeeinheit, die dazu konfiguriert ist, mindestens ein Ergebnis zu erzeugen, das eine Detektionswarnung umfasst, wenn der Prozessor bestimmt, dass

- der mindestens eine Belastbarkeitsschwellenwert erreicht wurde und der mindestens eine Schwellenwert für die zirkadiane Basalherzfrequenz erreicht wurde.

2. Detektionssystem nach Anspruch 1, wobei das mindestens eine Ergebnis eine Anfälligkeitswarnung umfasst, wenn der Prozessor bestimmt, dass der mindestens eine Belastbarkeitsschwellenwert für die körperliche Aktivität und/oder die geistige Aktivität erreicht wurde.

3. Detektionssystem nach Anspruch 1 oder 2, wobei die vergleichenden individuellen Niveaus des metabolischen Energieverbrauchs Niveaus sind, die aus vorhergehenden Zeitreihen der Herzfrequenzkomponenten erhalten wurden, bevorzugt über ein vorheriges Zeitfenster von etwa 2 bis 60 Tagen, weiter bevorzugt über einen Zeitraum von 10 bis 40 Tagen, insbesondere über einen Zeitraum von etwa einem Monat.

4. Detektionssystem nach einem der Ansprüche 1 bis 3, wobei das Vergleichen der aktuellen Belastbarkeit mit mindestens einem Belastbarkeitsschwellenwert für die körperliche Aktivität und die geistige Aktivität das Vergleichen der aktuellen Belastbarkeit mit mindestens einer vorhergehenden Belastbarkeit und das Bestimmen, ob der mindestens eine Belastbarkeitsschwellenwert für die körperliche Aktivität und die geistige Aktivität erreicht wurde, umfasst.

5. Detektionssystem nach Anspruch 4, wobei das Vergleichen der aktuellen Belastbarkeit mit der vorhergehenden Belastbarkeit das Subtrahieren mindestens einer vorhergehenden Belastbarkeit von der aktuellen Belastbarkeit und das Bestimmen, dass der mindestens eine Belastbarkeitsschwellenwert erreicht wurde, wenn die von der aktuellen Belastbarkeit subtrahierte, mindestens eine vorhergehende Belastbarkeit niedriger ist als der mindestens eine Belastbarkeitsschwellenwert, umfasst.

6. Detektionssystem nach Anspruch 4 oder 5, wobei der mindestens eine Belastbarkeitsschwellenwert einen **Langzeit-Belastbarkeitsschwellenwert** umfasst, wobei das Vergleichen der aktuellen Belastbarkeit mit mindestens einer vorhergehenden Belastbarkeit und das Bestimmen, ob der mindestens eine Belastbarkeitsschwellenwert erreicht wurde, das Vergleichen der aktuellen Belastbarkeit mit einer **durchschnittlichen vorhergehenden Belastbarkeit** einer Anzahl vorhergehender Zeitrahmen und das Bestimmen, ob der Langzeit-Belastbarkeitsschwellenwert erreicht wurde, umfasst, und wobei die Ausgabeeinheit ferner dazu konfiguriert ist, das Ergebnis zu erzeugen, wenn der Prozessor bestimmt, dass der Langzeit-Belastbarkeitsschwellenwert erreicht wurde.

7. Detektionssystem nach Anspruch 6, wobei der mindestens eine Belastbarkeitsschwellenwert ferner einen **Kurzzeit-Belastbarkeitsschwellenwert** umfasst, wobei das Vergleichen der aktuellen Belastbarkeit mit der vorhergehenden Belastbarkeit und das Bestimmen, ob der mindestens eine Belastbarkeitsschwellenwert erreicht wurde, ferner das Vergleichen der aktuellen Belastbarkeit mit der **unmittelbar vorhergehenden Belastbarkeit** eines unmittelbar vorhergehenden Zeitrahmens und das Bestimmen, ob der Kurzzeit-Belastbarkeitsschwellenwert erreicht wurde, umfasst, und wobei die Ausgabeeinheit ferner dazu konfiguriert ist, das Ergebnis zu erzeugen, wenn der Prozessor bestimmt, dass sowohl der Kurzzeit-Belastbarkeitsschwellenwert als auch der Langzeit-Belastbarkeitsschwellenwert erreicht wurden.

8. Detektionssystem nach einem der Ansprüche 1 bis 7, wobei der mindestens eine Prozessor ferner dazu programmiert ist, vergleichende individuelle Niveaus des metabolischen Energieverbrauchs für den zirkadianen Basalmetabolismus auf der Grundlage vorhergehender Zeitreihen der zirkadianen Basalherzfrequenz zur Verwendung als der mindestens eine Schwellenwert für die zirkadiane Basalherzfrequenz zu berechnen.

9. Detektionssystem nach einem der Ansprüche 1 bis 8, wobei der mindestens eine Prozessor ferner dazu programmiert ist, die Dynamik der zirkadianen Basalherzfrequenzkomponente zu analysieren, indem er die aktuelle zirkadiane Basalherzfrequenzkomponente mit mindestens einer vorhergehenden zirkadianen Basalherzfrequenzkomponente aus mindestens einem vorhergehenden Zeitrahmen vergleicht und bestimmt, ob der mindestens eine Schwellenwert für die zirkadiane Basalherzfrequenz erreicht wurde.

10. Detektionssystem nach Anspruch 9, wobei

- der mindestens eine Prozessor weiterhin programmiert ist, die Dynamik der zirkadianen Basalherzfrequenzkomponente zu analysieren, durch

▪ Vergleichen der aktuellen zirkadianen Basalherzfrequenzkomponente mit der **unmittelbar vorhergehenden zirkadianen Basalherzfrequenzkomponente** eines unmittelbar vorhergehenden Zeitrahmens und einem **Schwellenwert einer schnellen zirkadianen Basalherzfrequenz** des mindestens einen Schwellenwerts für die zirkadiane Basalherzfrequenz; und
▪ Vergleichen der aktuellen zirkadianen basalen Herzfrequenzkomponente mit einer durchschnittlichen vorhergehenden zirkadianen Basalherzfrequenzkomponente einer Anzahl von vorangehenden Zeitrahmen und einem **Schwellenwert einer langsamen zirkadianen Basalherzfrequenz** des mindestens einen Schwellenwerts für die zirkadiane Basalherzfrequenz;

- die Ausgabeeinheit ferner dazu konfiguriert ist, das Ergebnis zu erzeugen, wenn der Prozessor auch bestimmt, dass

▪ der Schwellenwert für die schnelle zirkadiane Basalherzfrequenz und der Schwellenwert für die langsame zirkadiane Basalherzfrequenz erreicht wurden.

11. Detektionssystem nach Anspruch 9 oder 10, wobei der mindestens eine Prozessor ferner dazu programmiert ist, die Dynamik der zirkadianen Basalherzfrequenzkomponente zu analysieren, indem er mit dem mindestens einen Schwellenwert vergleicht

▪ einen schnellen Trend der zirkadianen Basalherzfrequenzkomponente, der einer Änderung über einen kurzen Zeitraum von 1 bis 9 Tagen, bevorzugt 1 bis 7 Tagen, insbesondere 1 bis 2 Tagen, entspricht, und
▪ einen langsamen Trend der zirkadianen Basalherzfrequenzkomponente, der einer Veränderung über einen langen Zeitraum von 10 bis 40 Tagen, bevorzugt 10 bis 30 Tagen, insbesondere 20 bis 28 Tagen, entspricht.

12. Detektionssystem nach einem der Ansprüche 1 bis 11, wobei der mindestens eine Sensor einen Beschleunigungsmesser, ein Gyroskop, einen Bewegungssensor, ein GPS, eine Kamera, einen elektrischen Sensor, einen optischen Sensor, eine Elektrokardiogrammvorrichtung, einen Herzschallsensor, eine Laservorrichtung, einen Magnetfeldsensor, einen Schrittzähler und/oder einen Schallanalysator umfasst; und/oder wobei das Detektionssystem zumindest teilweise in eine tragbare Vorrichtung, wie eine Smartwatch, ein Smartphone, ein Brustband, ein Armband, ein Pflaster und/oder einen Aufkleber, integriert ist.

13. Detektionssystem nach einem der Ansprüche 1 bis 12, wobei die Belastbarkeit in einem Zeitrahmen von mindestens einem Tag berechnet wird und wobei die aktuelle Belastbarkeit die Belastbarkeit von mindestens dem aktuellen Tag ist.

14. Detektionssystem nach einem der Ansprüche 1 bis 13, wobei der Zeitrahmen einem Zeitraum von mindestens einem Tag entspricht und die Anzahl der vorhergehenden Zeitrahmen einem Gesamtzeitraum von 3 bis 60 Tagen, insbesondere einem Zeitraum von ungefähr einer Woche oder ungefähr einem Monat, entspricht.

15. Detektionssystem nach einem der Ansprüche 1 bis 14, wobei der mindestens eine Prozessor ferner programmiert ist,

- vergleichende individuelle Niveaus des metabolischen Energieverbrauchs für den zirkadianen Basalmetabolismus auf der Grundlage vorhergehender Zeitreihen der zirkadianen Basalherzfrequenzkomponente zu berechnen;
- aktuelle individuelle Niveaus des metabolischen Energieverbrauchs für den zirkadianen Basalmetabolismus auf der Grundlage der aktuellen zirkadianen Basalherzfrequenzkomponente zu berechnen;
- die Erholung zu bestimmen, wenn das aktuelle individuelle Niveau des metabolischen Energieverbrauchs niedriger ist als das vergleichende individuelle Niveau des metabolischen Energieverbrauchs für den zirkadianen Basalmetabolismus; und
- die Belastung zu bestimmen, wenn das aktuelle individuelle Niveau des metabolischen Energieverbrauchs höher ist als das vergleichende individuelle Niveau des metabolischen Energieverbrauchs für den zirkadianen Basalmetabolismus;

zum Klassifizieren der Detektionswarnung als eine Bakterieninfektionswarnung oder als eine Virusinfektionswarnung, wobei der mindestens eine Prozessor weiterhin programmiert ist,

- die Detektionswarnung als Bakterieninfektionswarnung zu klassifizieren, wenn der Prozessor bestimmt, dass der metabolische Energieverbrauch für den zirkadianen Basalmetabolismus zwischen 5 und 30 Tagen, bevorzugt zwischen 10 und 25 Tagen, vor der Detektionswarnung und zum Zeitpunkt der Detektionswarnung ansteigt; und/oder
- die Detektionswarnung als Virusinfektionswarnung zu klassifizieren, wenn der Prozessor bestimmt, dass die Erholung für den zirkadianen Basalmetabolismus zwischen 2 und 9 Tagen, bevorzugt zwischen 5 und 6 Tagen, vor der Detektionswarnung ansteigt und der metabolische Energieverbrauch für den zirkadianen Basalmetabolismus zwischen 5 und 15 Tagen, bevorzugt ungefähr 10 Tagen, nach der Detektionswarnung ansteigt;

wobei die Ausgabeeinheit ferner dazu konfiguriert ist, das mindestens eine Ergebnis zu erzeugen, das eine Virusinfektionswarnung und/oder eine Bakterieninfektionswarnung umfasst.

16. Computerlesbares Medium, das ein Computerprogramm und Anweisungen zum Durchführen eines Verfahrens zum Vorhersagen der Anfälligkeit und zum Erkennen einer Infektion und/oder Entzündung eines homöothermen lebenden Organismus speichert, wobei das Verfahren umfasst:

■ Messen und Überwachen, unter Verwendung mindestens eines Sensors, der Herzfrequenz und der körperlichen Aktivität des lebenden Organismus in Abhängigkeit der Zeit, um Zeitreihen der Herzfrequenz und der körperlichen Aktivität zu erhalten;
■ Zerlegen, unter Verwendung mindestens eines Prozessors, der Herzfrequenz in mindestens Zeitreihen einer körperlichen Herzfrequenzkomponente aufgrund körperlicher Aktivität ($HR_{physical}$), Zeitreihen einer geistigen Herzfrequenzkomponente aufgrund geistiger Aktivität ($HR_{mental}$) und Zeitreihen einer zirkadianen Basalherzfrequenzkomponente aufgrund des Basalmetabolismus ($HR_{circadian}$);
■ Berechnen, unter Verwendung des mindestens einen Prozessors, vergleichender individueller Niveaus des metabolischen Energieverbrauchs für die körperliche Aktivität und die geistige Aktivität auf der Grundlage vorhergehender Zeitreihen der Herzfrequenzkomponenten für die körperliche Aktivität bzw. die geistige Aktivität;
■ Berechnen, unter Verwendung des mindestens einen Prozessors, aktueller individueller Niveaus des metabolischen Energieverbrauchs für die körperliche Aktivität und die geistige Aktivität auf der Grundlage der aktuellen Herzfrequenzkomponenten;
■ Bestimmen, unter Verwendung des mindestens einen Prozessors, der Erholung für die körperliche Aktivität und die geistige Aktivität, wenn das aktuelle individuelle Niveau des metabolischen Energieverbrauchs niedriger ist als das vergleichende individuelle Niveau des metabolischen Energieverbrauchs;
■ Bestimmen, unter Verwendung des mindestens einen Prozessors, der Belastung für die körperliche Aktivität und die geistige Aktivität, wenn das aktuelle individuelle Niveau des metabolischen Energieverbrauchs höher ist als das vergleichende individuelle Niveau des metabolischen Energieverbrauchs;
■ Berechnen, unter Verwendung des mindestens einen Prozessors, der Belastbarkeit für die körperliche Aktivität und die geistige Aktivität auf der Grundlage des Verhältnisses zwischen der Erholung und der Belastung;
■ Vergleichen, unter Verwendung des mindestens einen Prozessors, der aktuellen Belastbarkeit für die körperliche Aktivität und die geistige Aktivität mit mindestens einem Belastbarkeitsschwellenwert;
■ Vergleichen, unter Verwendung des mindestens einen Prozessors, der aktuellen zirkadianen Basalherzfrequenzkomponente mit mindestens einem Schwellenwert für die zirkadiane Basalherzfrequenz;
■ Erzeugen eines Ergebnisses unter Verwendung einer Ausgabeeinheit, wenn der Prozessor bestimmt, dass der mindestens eine Belastbarkeitsschwellenwert und der mindestens eine Schwellenwert für die zirkadiane Basalherzfrequenz erreicht wurden.

## Revendications

1. Système de détection pour détecter la vulnérabilité à l'infection et/ou à l'inflammation et pour détecter une infection et/ou une inflammation d'un organisme vivant homéotherme, le système comprenant :

- au moins un capteur pour mesurer et surveiller en fonction du temps la fréquence cardiaque ainsi que l'activité physique de l'organisme vivant dans le but d'obtenir des séries temporelles de la fréquence cardiaque et de l'activité physique ;
- au moins un processeur programmé pour

- décomposer la fréquence cardiaque en au moins une série temporelle d'une composante de la fréquence cardiaque physique due à l'activité physique ($HR_{physique}$), une série temporelle d'une composante de la fréquence cardiaque mentale due à l'activité mentale ($HR_{mentale}$), et une série temporelle d'une composante circadienne de la fréquence cardiaque de base due au métabolisme circadien de base ($HR_{circadienne}$) ;
- calculer des niveaux individuels comparatifs de dépense énergétique métabolique pour l'activité physique et pour l'activité mentale sur la base des séries temporelles précédentes, respectivement, d'au moins la composante physique et d'au moins la composante mentale de la fréquence cardiaque ;
- calculer des niveaux individuels actuels de dépense énergétique métabolique pour l'activité physique et pour l'activité mentale sur la base des composantes actuelles de la fréquence cardiaque ;
- déterminer la récupération lorsque le niveau individuel actuel de dépense énergétique métabolique est plus bas que le niveau individuel comparatif de dépense énergétique métabolique pour l'activité physique ainsi que pour l'activité mentale ;
- déterminer la charge lorsque le niveau individuel actuel de dépense énergétique métabolique est plus élevé que le niveau individuel comparatif de dépense énergétique métabolique pour l'activité physique ainsi que pour l'activité mentale ;
- calculer la résilience sur la base du rapport entre la récupération et la charge pour l'activité physique ainsi que pour l'activité mentale ;
- comparer la résilience actuelle avec au moins un seuil de résilience pour l'activité physique et pour l'activité mentale et déterminer si ledit au moins un seuil de résilience a été atteint pour l'activité physique et/ou pour l'activité mentale ;
- comparer la composante circadienne actuelle de la fréquence cardiaque de base avec au moins un seuil de la fréquence cardiaque circadienne de base et déterminer si ledit au moins un seuil de la fréquence cardiaque circadienne de base a été atteint ;

- une unité de sortie configurée de manière à générer au moins un résultat comprenant un avertissement de détection lorsque le processeur détermine que

- ledit au moins un seuil de résilience a été atteint et que ledit au moins un seuil de la fréquence cardiaque circadienne de base a été atteint.

2. Système de détection selon la revendication 1, dans lequel ledit au moins un résultat comprend un avertissement de vulnérabilité lorsque le processeur détermine que ledit au moins un seuil de résilience a été atteint pour l'activité physique et/ou pour l'activité mentale.

3. Système de détection selon la revendication 1 ou 2, dans lequel les niveaux individuels comparatifs de dépense énergétique métabolique sont des niveaux obtenus à partir de séries temporelles précédentes des composantes de la fréquence cardiaque, de préférence sur une fenêtre temporelle antérieure d'environ 2 à 60 jours, mieux encore sur une période de 10 à 40 jours, en particulier sur une période d'environ un mois.

4. Système de détection selon l'une quelconque des revendications 1 à 3, dans lequel la comparaison de la résilience actuelle avec au moins un seuil de résilience pour l'activité physique et pour l'activité mentale consiste à comparer la résilience actuelle avec au moins une résilience précédente et à déterminer si ledit au moins un seuil de résilience a été atteint pour l'activité physique ainsi que pour l'activité mentale.

5. Système de détection selon la revendication 4, dans lequel la comparaison de la résilience actuelle avec la résilience précédente consiste à soustraire au moins une résilience précédente de la résilience actuelle et à déterminer que ledit au moins un seuil de résilience a été atteint lorsque ladite au moins une résilience précédente soustraite de la résilience actuelle est inférieure audit au moins un seuil de résilience.

6. Système de détection selon la revendication 4 ou 5, dans lequel ledit au moins un seuil de résilience comprend un seuil de résilience à long terme, dans lequel la comparaison de la résilience actuelle avec au moins une résilience précédente et la détermination que ledit au moins un seuil de résilience a été atteint comprennent la comparaison de la résilience actuelle avec une résilience précédente moyenne d'un certain nombre de périodes de temps précédentes ainsi que la détermination que le seuil de résilience à long terme a été atteint, et dans lequel l'unité de sortie est en outre configurée de manière à générer le résultat lorsque le processeur détermine que le seuil de résilience à long terme a été atteint.

7. Système de détection selon la revendication 6, dans lequel ledit au moins un seuil de résilience comprend en outre un seuil de résilience à court terme, dans lequel la comparaison de la résilience actuelle avec la résilience précédente et la détermination que ledit au moins un seuil de résilience a été atteint comprennent en outre la comparaison de la résilience actuelle avec la résilience immédiatement précédente d'une période de temps immédiatement précédente ainsi que la détermination que le seuil de résilience à court terme a été atteint, et dans lequel l'unité de sortie est en outre configurée de manière à générer le résultat lorsque le processeur détermine que le seuil de résilience à court terme et le seuil de résilience à long terme ont tous les deux été atteints.

8. Système de détection selon l'une quelconque des revendications 1 à 7, dans lequel ledit au moins un processeur est en outre programmé pour calculer des niveaux individuels comparatifs de dépense énergétique métabolique pour le métabolisme circadien de base sur la base des séries temporelles précédentes de la fréquence cardiaque circadienne de base, fréquence cardiaque à utiliser en tant que ledit au moins un seuil de la fréquence cardiaque circadienne de base.

9. Système de détection selon l'une quelconque des revendications 1 à 8, dans lequel ledit au moins un processeur est en outre programmé pour analyser la dynamique de la composante circadienne de la fréquence cardiaque de base en comparant la composante circadienne actuelle de la fréquence cardiaque de base avec au moins une composante circadienne précédente de la fréquence cardiaque de base d'au moins une période de temps précédente et en déterminant que ledit au moins un seuil de la fréquence circadienne de base a été atteint.

10. Système de détection selon la revendication 9, dans lequel

- ledit au moins un processeur est en outre programmé pour analyser la dynamique de la composante circadienne de la fréquence cardiaque de base en

  ■ comparant la composante actuelle de la fréquence cardiaque circadienne de base avec la composante circadienne immédiatement précédente de la fréquence cardiaque de base d'une période de temps immédiatement précédente et avec un seuil de la fréquence cardiaque circadienne de base rapide dudit au moins un seuil de la fréquence cardiaque circadienne de base ; et en
  ■ comparant la composante circadienne actuelle de la fréquence cardiaque de base avec une composante circadienne précédente moyenne de la fréquence cardiaque de base d'un certain nombre de périodes de temps précédentes et avec un seuil de la fréquence cardiaque circadienne de base lente dudit au moins un seuil de la fréquence cardiaque circadienne de base ;

- dans lequel l'unité de sortie est en outre configurée de manière à générer le résultat lorsque le processeur détermine également que

  ■ le seuil de la fréquence cardiaque circadienne de base rapide et le seuil de la fréquence cardiaque circadienne de base lente ont été atteints.

11. Système de détection selon la revendication 9 ou 10, dans lequel ledit au moins un processeur est en outre programmé pour analyser la dynamique de la composante circadienne de la fréquence cardiaque de base en comparant celle-ci avec ledit au moins un seuil,

  ■ une tendance rapide de la composante circadienne de la fréquence cardiaque de base correspondant à une variation sur une période courte de 1 à 9 jours, de préférence 1 à 7 jours, en particulier 1 à 2 jours, et
  ■ une tendance lente de la composante circadienne de la fréquence cardiaque de base correspondant à une variation sur une période longue de 10 à 40 jours, de préférence de 10 à 30 jours, en particulier de 20 à 28 jours.

12. Système de détection selon l'une quelconque des revendications 1 à 11, dans lequel ledit au moins un capteur comprend un accéléromètre, un gyroscope, un capteur de mouvement, un GPS, une caméra, un capteur électrique, un capteur optique, un dispositif d'électrocardiogramme, un capteur de bruits cardiaques, un laser, un capteur de champ magnétique, un podomètre et/ou un analyseur de sons ; et/ou dans lequel le système de détection est au moins partiellement intégré à l'intérieur d'un dispositif portable tel qu'une montre intelligente, un téléphone intelligent, une bande pectorale, un bracelet, un patch et/ou un autocollant.

**13.** Système de détection selon l'une quelconque des revendications 1 à 12, dans lequel la résilience est calculée sur une période de temps d'au moins un jour, et dans lequel la résilience actuelle est la résilience d'au moins la journée en cours.

**14.** Système de détection selon l'une quelconque des revendications 1 à 13, dans lequel la période de temps correspond à une période d'au moins un jour, et le nombre de périodes de temps précédentes correspond à une période totale de 3 à 60 jours, en particulier à une période d'environ une semaine ou d'environ un mois.

**15.** Système de détection selon l'une quelconque des revendications 1 à 14, dans lequel ledit au moins un processeur est en outre programmé pour

- calculer des niveaux individuels comparatifs de dépense énergétique métabolique pour le métabolisme circadien de base sur la base des séries temporelles précédentes de la composante circadienne de la fréquence cardiaque de base ;
- calculer des niveaux individuels actuels de dépense énergétique métabolique pour le métabolisme circadien de base sur la base de la composante circadienne actuelle de la fréquence cardiaque de base ;
- déterminer la récupération lorsque le niveau individuel actuel de dépense énergétique métabolique est plus bas que le niveau individuel comparatif de dépense énergétique métabolique pour le métabolisme circadien de base ; et
- déterminer la charge lorsque le niveau individuel actuel de dépense énergétique métabolique est plus élevé que le niveau individuel comparatif de dépense énergétique métabolique pour le métabolisme circadien de base ;

afin de classer l'avertissement de détection comme un avertissement d'infection bactérienne ou comme un avertissement d'infection virale, ledit au moins un processeur étant en outre programmé pour

- classer l'alerte de détection comme une alerte d'infection bactérienne lorsque le processeur détermine que la dépense d'énergie métabolique pour le métabolisme circadien de base augmente entre 5 et 30 jours, de préférence entre 10 et 25 jours, avant l'alerte de détection et au moment de l'alerte de détection ; et/ou
- classer l'alerte de détection comme une alerte d'infection virale lorsque le processeur détermine que la récupération du métabolisme circadien de base augmente entre 2 et 9 jours, de préférence entre 5 et 6 jours, avant l'alerte de détection et que la dépense d'énergie métabolique pour le métabolisme circadien de base augmente entre 5 et 15 jours, de préférence environ 10 jours, après l'alerte de détection ;

dans lequel l'unité de sortie est en outre configurée de manière à générer au moins un résultat comprenant un avertissement d'infection virale et/ou un avertissement d'infection bactérienne.

**16.** Support lisible par un ordinateur qui stocke un programme informatique ainsi que des instructions pour exécuter un procédé de prédiction de la vulnérabilité et pour détecter une infection et/ou une inflammation d'un organisme vivant homéotherme, le procédé comprenant les étapes suivantes :

- mesurer et surveiller, en utilisant au moins un capteur, la fréquence cardiaque ainsi que l'activité physique de l'organisme vivant en fonction du temps, dans le but d'obtenir des séries temporelles de la fréquence cardiaque et de l'activité physique ;
- décomposer, en utilisant au moins un processeur, la fréquence cardiaque en au moins une série temporelle d'une composante de la fréquence cardiaque physique due à l'activité physique ($HR_{physique}$), une série temporelle d'une composante de la fréquence cardiaque mentale due à l'activité mentale ($HR_{mentale}$), et une série temporelle d'une composante circadienne de la fréquence cardiaque de base due au métabolisme de base ($HR_{circadienne}$) ;
- calculer, en utilisant ledit au moins un processeur, des niveaux individuels comparatifs de dépense énergétique métabolique pour l'activité physique et pour l'activité mentale, sur la base des séries temporelles précédentes des composantes de la fréquence cardiaque pour l'activité physique et, respectivement, pour l'activité mentale ;
- calculer, en utilisant ledit au moins un processeur, des niveaux individuels actuels de dépense énergétique métabolique pour l'activité physique et pour l'activité mentale sur la base des composantes actuelles de la fréquence cardiaque ;
- déterminer, en utilisant ledit au moins un processeur, la récupération de l'activité physique et de l'activité mentale lorsque le niveau individuel actuel de dépense énergétique métabolique est plus bas que le niveau individuel comparatif de dépense énergétique métabolique ;

- déterminer, en utilisant ledit au moins un processeur, la charge de l'activité physique et de l'activité mentale lorsque le niveau individuel actuel de dépense énergétique métabolique est plus élevé que le niveau individuel comparatif de dépense énergétique métabolique ;
- calculer, en utilisant ledit au moins un processeur, la résilience pour l'activité physique et pour l'activité mentale sur la base du rapport entre la récupération et la charge ;
- comparer, en utilisant ledit au moins un processeur, la résilience actuelle pour l'activité physique et pour l'activité mentale avec au moins un seuil de résilience ;
- comparer, en utilisant ledit au moins un processeur, la composante circadienne actuelle de la fréquence cardiaque de base avec au moins un seuil de la fréquence cardiaque circadienne de base ;
- générer un résultat en utilisant une unité de sortie lorsque le processeur détermine que ledit au moins un seuil de résilience et que ledit au moins un seuil de la fréquence cardiaque circadienne de base ont été atteints.

FIG. 1

measure **heart rate** and **physical activity**

decompose heart rate in **physical heart rate component, mental heart rate component** and **circadian basal heart rate component**

calculate **comparative individual level of metabolic energy use** for the physical and for the mental component

determine **load** and **recovery** for the physical and for the mental components

calculate **resilience** for the physical and for the mental components

compare resilience with **individual resilience threshold** and circadian basal heart rate with **individual circadian basal heart rate threshold**

**alert for vulnerability** (vulnerability warning / prediction alert) when resilience threshold has been reached and **alert for infection** (detection warning / detection alert) when resilience threshold and circadian basal heart rate threshold have been reached

FIG. 2

FIG. 3

I.e. Sensors
in bracelet

$HR_{Total}$

Movement

In watch/phone
real-time
algorithm

$HR_{Circadian}$

$HR_{Physical}$

Encrypted and anonymized

FIG. 4      $HR_{Mental} = HR_{Total} - HR_{Physical} - HR_{Circadian}$

$mnt_{avg}(t-1)$    $mnt_{var}(t-1)$    $mnt_n(t-1)$    $\hat{y}_{physical}$

States    Parameters

$$mnt_{avg} = b_0 * States[8] - a_1 * mnt_{avg}(t-1)$$
$$mnt_{var} = mnt_{var} + \left(States[8] - mnt_{avg}(t-1)\right)^2$$
$$mnt_{std} = \sqrt{mnt_{var}(t-1)/(mnt_n+1)}$$
$$mnt_n = mnt_n + 1$$

$$amp = \sqrt{States[2]^2 + States[3]^2}$$

$$\hat{y}_{mental} = States[8] - mnt_{avg} + 1{,}5 * mnt_{std}$$

$$\hat{y}_{circadian} = amp + Parameters[2:3] * States[2:3]$$

$$\hat{y}_{basal} = States[1] - amp + mnt_{avg} - 1{,}5 * mnt_{std}$$

FIG. 5

HR<sub>Total</sub> [bpm]

HR<sub>Circadian</sub> [bpm]

HR<sub>Physical</sub> [bpm]

HR<sub>Mental</sub> [bpm]

FIG. 6

FIG. 7

FIG. 8

FIG. 9

FIG. 10

FIG. 11

FIG. 12

FIG. 13

$$mnt_{avg} \qquad mnt_{std} \qquad y_{non\ physical}$$

$$sr = 100 * \frac{y_{non_{physical}} - mnt_{avg}}{3 * mnt_{std}}$$

Ment_Load = max(0, min(100,sr))
Ment_Recv = max(0, min(100,-sr))

FIG. 14

$$phys_{med} \qquad phys_{std} \qquad y_{physical}$$

$$lr = 100 * \frac{y_{physical} - phys_{med}}{1.5 * phys_{std}}$$

Phys_Load = max(0, min(100,lr))
Phys_Recv = max(0, min(100,-lr))

FIG. 15

Mental energy Use and Recovery

Physical energy Load and Recovery

FIG. 16

Time
Phys_Load
Phys_Recv
Ment_Load
Ment_Recv          FIG. 17

① Resilience

② Prediction

Alert

Time
Phys_Load
Phys_Recv
Ment_Load
Ment_Recv

t_1 = 00:00; t_2 = 23:59

MP_Load(t) = sum(Phys_Load(t1:t2,1))
MP_Recv(t) = sum(Phys_Recv(t1:t2,1))

t_1 = 00:00; t_2 = 23:59

ME_Use(t) = sum(Ment_Load(t1:t2,1))
ME_Recv(t) = sum(Ment_Recv(t1:t2,1))

FIG. 18

$$Res_{phys}(t) = \frac{MP\_Recv}{MP\_Load}$$

$$Res_{ment}(t) = \frac{ME\_Recv}{ME\_Use}$$

$Res_{phys}$

$Res_{Ment}$

$$Prediction_{Phys} = Res_{Phys}(t) \leq 1.0$$

$$Prediction_{Ment} = Res_{Ment}(t) \leq 1.0$$

$Prediction_{Phys}$

$Prediction_{Ment}$

FIG. 19

Alert

$Res_{phys}$

$Res_{Ment}$

$$Short_{phys}(t) = Res_{phys}(t) - Res_{phys}(t-1) \leq -5.0$$

$$Long_{phys}(t) = Res_{phys}(t) - Res_{phys}(t-7) \leq -10.0$$

$$Short_{Ment}(t) = Res_{Ment}(t) - Res_{Ment}(t-1) \leq -5.0$$

$$Long_{Ment}(t) = Res_{Ment}(t) - Res_{Ment}(t-7) \leq -15.0$$

$$Prediction_{Phys} = Short_{phys}(t)\&\& /||Long_{phys}(t)$$

$$Prediction_{Ment} = Short_{ment}(t)\&\& /||Long_{ment}(t)$$

$Prediction_{Phys}$

$Prediction_{Ment}$

$Alert$

**FIG. 20**

**FIG. 21**

FIG. 22

FIG. 23

ME$_{recov}$

ME$_{load}$

**FIG. 25**

ME$_{recov}$

ME$_{load}$

**FIG. 26**

Energy use versus Recovery

Daily mental recovery and energy use

physical

Monthly overview of mental

Only 46 % recovery of used metabolic energy is an orange day in the monthly overview.

**FIG. 24**

BioRICS Mindstretch

For Preventive Health Management

STRESS
BURNOUT...

PERFORMANCE, INFECTION, DEPRESSION,

Automated & continuous Monitoring

Automated
Feedback

Subject in
daily operation

Evaluate
solution

Preventive healthcare:
- Self Management
- Advice
- Coaching

**FIG. 27**

FIG. 28

FIG. 29

FIG. 30

FIG. 31

FIG. 32

FIG. 33

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008003148 A

- BE 2007000075 W

**Non-patent literature cited in the description**

- **H. PYLORI. ; AZABO S.** Hans Selye 70 years later: steroids, stress ulcers. *Clin. Neuroscience,* 2014, vol. 69, 85-93
- **SZABO S. ; YOSHIDA M. ; FILAKOVSZKY J ; JUHASZ G.** Stress is 80 years old: from Hans Selye original paper in 1936 to recent advances in GI Ulceration. *Current Pharmaceutical Design,* 2014, vol. 23, 4029-4041
- **NORTON, T ; PIETTE, D. ; EXADAKTYLOS, V. ; BERCKMANS, D.** Automated real-time stress monitoring of police horses using wearable technology. *APPLIED ANIMAL BEHAVIOUR SCIENCE,* 2018, vol. 198, 67-74
- **PIETTE D. ; NORTON T. ; VRIEZE E. ; BERCKMANS D. ; AERTS J.M. ; EXADAKTYLOS V. ; BOGAERTS K. ; HOMPES T. ; CLAES S.** Dynamic modelling of the cardiovascular response to a combined physical and mental task to identify biomarkers for psychopathology. *Psychological Medicine*
- **RUSSELL A. ; HENEGHAN C. ; VENKATRAMAN S.** Investigation of an estimate of daily resting heart rate using a consumer wearable device. *medRxiv,* 18 October 2019, 1-10, https://doi.org/10.1101/19008771doi

- **JENNIFER M RADIN ; NATHAN E WINEINGER ; ERIC J. TOPOL ; STEVEN R STEINHUBL.** Harnessing wearable device data to improve state-level real-time surveillance of influenza-like illness in the USA: a population-based study. *The Lancet.com/digital-health,* 2020, e85-393
- **BRAGE S ; BRAGE N ; FRANKS PW ; EKELUND U ; WAREHAM NJ.** Reliability and validity of the combined heart rate and movement sensor Actiheart. *Eur J Clin Nutr,* 2005, vol. 59, 561-570
- **SEGERSTROM, S.C.** Resources, Stress, and Immunity: An Ecological Perspective on Human Psychoneuroimmunology. *ANNALS OF BEHAVIORAL MEDICINE,* 2010, vol. 40 (1), 114-125
- **SEGERSTROM, S.C. ; MILLER, G.E.** Psychological stress and the human immune system: A meta-analytic study of 30 years of inquiry. *PSYCHOLOGICAL BULLETIN,* 2004, vol. 130 (4), 601-630
- **SELYE H.** The stress of life. Mc Graw Hill, 1975
- **FOWLER G.A.** Wearable tech can spot coronavirus symptoms before you even realize you're sick. *Technology columnist,* 2020, 202